(19) Europäisches Patentamt
European Patent Office
Office européen des brevets

(11) Veröffentlichungsnummer : **0 049 814**
**B1**

(12) # EUROPÄISCHE PATENTSCHRIFT

(45) Veröffentlichungstag der Patentschrift : **09.01.85**

(51) Int. Cl.[4] : **C 07 D501/36**, C 07 D501/57, A 61 K 31/545

(21) Anmeldenummer : **81107752.8**

(22) Anmeldetag : **30.09.81**

(54) **Neue Cephalosporine, Verfahren zu ihrer Herstellung und diese Verbindungen enthaltende Arzneimittel.**

(30) Priorität : **11.10.80 DE 3038501**

(43) Veröffentlichungstag der Anmeldung : **21.04.82 Patentblatt 82/16**

(45) Bekanntmachung des Hinweises auf die Patenterteilung : **09.01.85 Patentblatt 85/02**

(84) Benannte Vertragsstaaten : **AT BE CH DE FR IT LI LU NL SE**

(56) Entgegenhaltungen :
**EP-A- 0 021 176**
**EP-A- 0 022 494**
**EP-A- 0 035 161**

(73) Patentinhaber : **Dr. Karl Thomae GmbH**
**Postfach 1755**
**D-7950 Biberach (Riss) (DE)**

(72) Erfinder : **Wetzel, Bernd, Dr., Dipl.-Chem**
**Kapellenweg 21**
**D-7950 Biberach 1 (DE)**
Erfinder : **Woitun, Eberhard, Dr., Dipl.-Chem.**
**Stecherweg 17**
**D-7950 Biberach 1 (DE)**
Erfinder : **Reuter, Wolfgang, Dr., Dipl.-Chem.**
**Nelkenweg 10**
**D-7951 Laupertshausen (DE)**
Erfinder : **Maier, Roland, Dr., Dipl.-Chem.**
**Bodelschwinghstrasse 39**
**D-7950 Biberach 1 (DE)**
Erfinder : **Lechner, Uwe, Dr.**
**Panoramastrasse 12**
**D-7951 Ummendorf (DE)**
Erfinder : **Goeth, Hanns, Dr.**
**Oberer Bühl 6**
**D-7950 Biberach 1 (DE)**

EP 0 049 814 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Cephalosporine der allgemeinene Formel I

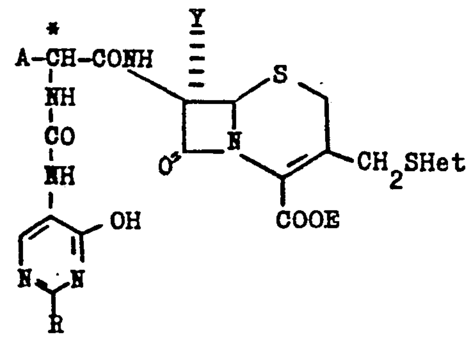

(I)

Verfahren zur Herstellung dieser Verbindungen und diese Verbindungen enthaltende Arzneimittel.

In der allgemeinen Formel I bedeuten :

A die Phenyl-, 4-Hydroxyphenyl-, 2-Thienyl-, 2-Furylgruppe, oder einen 3,4-Dihydroxyphenylrest,

Y ein Wasserstoffatom oder die Methoxygruppe,

Het die 4H-5,6-Dioxo-1,2,4-triazin-3-yl-, die 4-Methyl-5,6-dioxo-1,2,4-triazin-3-yl-, die 2-Methyl-5,6-dioxo-1,2,4-triazin-3-yl, die 1-Vinyl-tetrazol-5-yl- oder 1-Allyl-tetrazol-5-yl-gruppe oder eine Gruppe der allgemeinen Formel

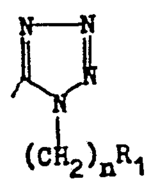

wobei n die Zahlen 1 bis 3 bedeutet und $R_1$ für die Hydroxygruppe, die Amino-, Dimethylamino-, Acetylamino-, Aminocarbonyl-, Aminocarbonylamino-, Aminosulfonyl-, Aminosulfonylamino-, Methylcarbonyl-, Methylsulfonylamino-, Cyano-, Hydroxysulfonylamino-, Methylsulfonyl-, Methylsulfinyl-, sowie für eine Carbonsäure oder Sulfonsäuregruppe steht, weiter kann $(CH_2)_nR_1$ eine Alkylgruppe mit 2 bis 4 Kohlenstoffatomen oder einen 2,3-Dihydroxypropylrest bedeuten,

E ein Wasserstoffatom oder eine in vitro oder in vivo leicht spaltbare Schutzgruppe und

R die Cyclopropylgruppe oder eine Gruppe der allgemeinen Formel

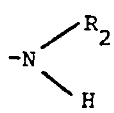 ,

worin $R_2$ eine verzweigte oder unverzweigte, gesättigte oder ungesättigte Alkylgruppe mit 1 bis 4 Kohlenstoffatomen oder einen Cycloalkylrest mit 3 bis 6 Kohlenstoffatomen bedeutet,

R bedeutet weiter eine Gruppe der allgemeinen Formel —NH—Z—G, bei der —NH—Z— eine Gruppe der Formeln

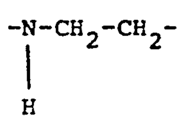 oder 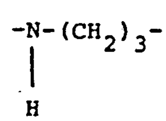

darstellt, und G die Hydroxy-, Aminocarbonyl-, Aminosulfonyl-, Aminocarbonylamino-, Acetylamino-, Methylsulfonylamino-, Methylsulfinyl- oder Methylsulfonylgruppe bedeutet oder in der

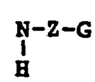

die 4′-Hydroxycyclohexylaminogruppe bedeutet ;

R bedeutet weiter eine Gruppe der allgemeinen Formel

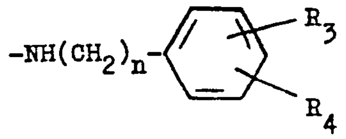

worin n = 0 oder 1 und $R_3$ und $R_4$, die gleich oder verschieden sein können, Wasserstoffatome, Chlor- oder Fluoratome, eine Hydroxy-, Methoxy-, Acetylamino-, Aminocarbonylamino-, Nitro-, Acetyl-, Methylcarbonyloxy-, Methoxycarbonyl-, Aminocarbonyl-, Cyan-, Methylsulfinyl-, Methylsulfonyl-, Aminosulfonyl-, Methylaminosulfonyl- oder eine Methylgruppe bedeuten.

Als Carboxylschutzgruppen E kommen im Sinne der Erfindung solche in Frage, welche auch bisher schon auf dem Gebiet der Penicilline und Cephalosporine eingesetzt wurden, insbesondere esterbildende Gruppen, die durch Hydrogenolyse oder Hydrolyse oder andere Behandlungen unter milden Bedingungen entfernt werden können oder esterbildende Gruppen, welche leicht im lebenden Organismus abgespalten werden können. Beispiele für in vitro leicht spaltbare Schutzgruppen sind z. B. die Benzyl-, Diphenylmethyl-, Trityl-, t-Butyl-, die 2,2,2-Trichloräthyl- oder die Trimethylsilylgruppe.

Bedeutet E Wasserstoff, so fallen unter den Anspruch auch pharmazeutisch verträgliche Salze, wie z. B. Alkali- oder Erdalkalisalze, z. B. Natrium-, Kalium-, Magnesium- oder Calciumsalze, Ammoniumsalze, organische Aminsalze, z. B. mit Triäthylamin oder Dicyclohexylamin.

Eine besonders bevorzugte Ausführungsform der Erfindung besteht darin, daß R die Gruppe der allgemeinen Formel

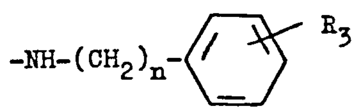

bedeutet, worin n = 0 oder 1 ist und $R_3$ Wasserstoffatom, die Hydroxy-, Nitro-, Acetylamino-, Methylsulfinyl-, Methylsulfonyl-, Acetyl-, Aminocarbonyl-, Aminocarbonylamino-, Aminosulfonyl- oder Methylaminosulfonylgruppe bedeutet, oder daß R die p-Aminosulfonyl-m-hydroxy-anilinogruppe oder die Cyclopropyl-, Propylamino-, Isopropylamino-, Cyclopentylamino-, Cyclohexylamino-, 3'-Hydroxy-propylamino-, 4'-Hydroxycyclohexylamino- oder 2'-Aminosulfonyläthylaminogruppe darstellt, E für Wasserstoff steht, und A, Y und Het wie vorstehend definiert sind.

Die Cephalosporinverbindungen der allgemeinen Formel I können in zwei tautomeren Formen (nämlich vom Lactim- und vom Lactamtyp) vorliegen. Es hängt besonders vom jeweiligen Lösemittel und von der Art des Substituenten R ab, welche der beiden Formen I oder I' überwiegt :

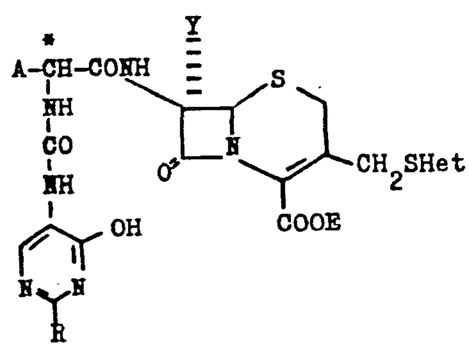

(I)

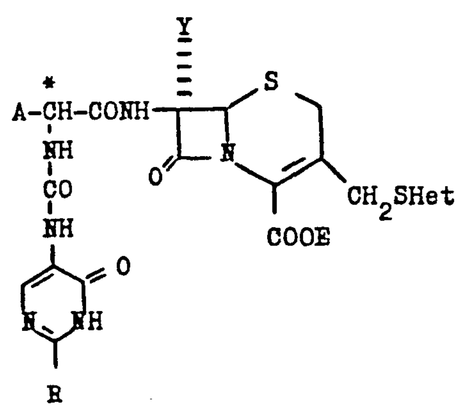

(I')

Es versteht sich von selbst, daß die eingangs angegebenen Verbindungen der allgemeinen Formel I immer beide Tautomeren umfassen.

Die Verbindungen der allgemeinen Formel I können bezüglich des Chiralitätszentrums $C^+$ in den beiden möglichen R- und S-Konfigurationen, jedoch auch als ein Gemisch dieser beiden Konfigurationen, vorliegen. Besonders bevorzugt sind solche Verbindungen, für welche die D = R-Konfiguration zutrifft. Wenn das Endprodukt in der D,L-Form anfällt, gelingt es, die reinen D- und L-Diastereomeren durch präparative Flüssigkeitschromatographie (HPLC) herzustellen.

Die Verbindungen der allgemeinen Formel I können wie folgt hergestellt werden :

1. Durch Umsetzung einer Verbindung der allgemeinen Formel

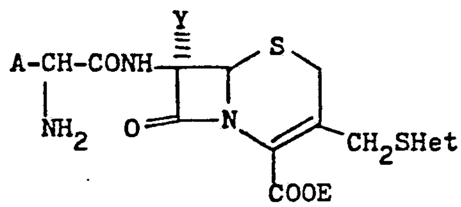

(II)

in der A, Y, E und Het die oben angegebenen Bedeutungen haben, mit einem Pyrimidinderivat der allgemeinen Formel III,

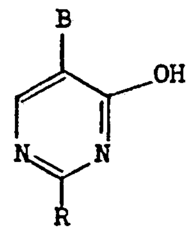

(III)

in der R wie oben definiert ist und B die Gruppe —NCO oder ein reaktives Derivat der Gruppe —NHCOOH bedeutet, wie z. B. die Gruppen —NHCOCl, —NHCOBr oder

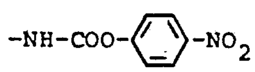

wobei die Gruppen —NCO und —NHCOCl besonders bevorzugt sind. Es können auch Gemische von solchen Pyrimidinderivaten der allgemeinen Formel III verwendet werden, in der B teils die eine und teils die andere der vorstehend genannten Bedeutungen besitzt, z. B. die Gruppen —NCO und

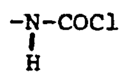

gleichzeitig nebeneinander.

Bedeutet E ein Wasserstoffatom, so können die Ausgangsprodukte der allgemeinen Formel II in Form ihrer anorganischen oder organischen Salze, z. B. als Triäthylammoniumsalz oder Natriumsalz, eingesetzt werden. Die Reaktion kann dann in beliebigen Mischungen von Wasser mit solchen organischen Lösungsmitteln, die mit Wasser mischbar sind, wie Ketonen, z. B. Aceton, cyclische Äther, z. B. Tetrahydrofuran oder Dioxan, Nitrilen, z. B. Acetonitril, Formamiden, z. B. Dimethylformamid, Dimethylsulfoxid oder Alkoholen, z. B. Isopropanol, oder in Hexametapol durchgeführt werden. Dabei hält man den pH der Reaktionsmischung durch Zusatz von Basen oder Verwendung von Pufferlösungen in einem pH-Bereich von etwa 2,0 bis 9,0, vorzugsweise zwischen pH 6,5 und 8,0. Es ist aber auch möglich, die Reaktion in wasserfreien organischen Lösungsmitteln, z. B. halogenierten Kohlenwasserstoffen, wie Chloroform oder Methylenchlorid, unter Zusatz von Basen, vorzugsweise Triäthylamin, Diäthylamin oder N-Äthylpiperidin, durchzuführen. Weiterhin läßt sich die Reaktion in einem Gemenge aus Wasser und einem mit Wasser nicht mischbaren Lösungsmittel, wie Äther, z. B. Diäthyläther, halogenierten Kohlenwasserstoffen, z. B. Chloroform oder Methylenchlorid, Schwefelkohlenstoff, Ketonen, z. B. Isobutylmethylketon, Estern, z. B. Essigsäureäthylester, aromatischen Lösungsmitteln, z. B. Benzol, ausführen, wobei es zweckmäßig ist, kräftig zu rühren, und den pH-Wert durch Basenzusatz oder Verwendung von Pufferlösungen in einem Bereich von etwa pH 2,0 bis 9,0, vorzugsweise zwischen 6,5 und 8,0, zu halten. Man kann die Reaktion aber auch in Wasser allein in Gegenwart einer organischen oder anorganischen Base oder unter Zusatz von Pufferstoffen durchführen.

Bedeutet E die Trimethylsilylgruppe, d. h. verwendet man als Ausgangsprodukte für das erfindungsgemäße Verfahren die Silylderivate der Verbindungen der allgemeinen Formel II (z. B. Mono- oder zweckmäßigerweise die an der Amino- und Carboxylgruppe silylierten Di-trimethylsilylderivate) und setzt sie mit Verbindungen der allgemeinen Formel III um, so arbeitet man im allgemeinen zweckmäßigerweise in wasser- und hydroxyl-gruppen-freien Lösungsmitteln, beispielsweise in halogenierten Kohlenwasserstoffen, z. B. Methylenchlorid oder Chloroform, Benzol, Tetrahydrofuran, Aceton oder Dimethylformamid usw. Der Zusatz von Basen ist hierbei nicht notwendig, kann jedoch einzelnen Fällen vorteilhaft sein, um die Ausbeute und Reinheit der Produkte zu verbessern. Als gegebenenfalls zugesetzte Basen werden zweckmäßigerweise tertiäre aliphatische oder aromatische Amine, wie Pyridin oder Triäthylamin, oder durch sterische Hinderung schweracylierbare sekundäre Amine, wie Dicyclohexylamin, benützt.

Bedeutet E eine der anderen oben genannten in vitro oder in vivo leicht spaltbaren Schutzgruppen, z. B. die Diphenylmethylgruppe oder die Pivaloyloxymethylgruppe, so empfiehlt es sich ebenfalls in einem aprotischen Lösungsmittel zu arbeiten, z. B. in absolutem Methylenchlorid, Chloroform, Tetrahydrofuran oder Dimethylformamid.

Die Menge der verwendeten Basen ist z. B. durch die gewünschte Einhaltung eines bestimmten pH-Wertes festgelegt. Wo eine pH-Messung und Einstellung nicht erfolgt oder wegen des Fehlens von ausreichenden Mengen Wasser im Verdünnungsmittel nicht möglich oder nicht sinnvoll ist, werden im Falle der Verwendung der nichtsilylierten Verbindungen der allgemeinen Formel II vorzugsweise 1,0 bis 2,0 Moläquivalente Basen eingesetzt. Im Falle der Verwendung der silylierten Verbindungen verwendet man vorzugsweise bis zu einem Moläquivalent Base.

Als Basen können prinzipiell alle in der organischen Chemie üblicherweise verwendeten organischen und anorganischen Basen verwendet werden, wie Alkali- und Erdalkalihydroxide, Erdalkalioxide, Alkali- und Erdalkalicarbonate und Hydrogencarbonate, Ammoniak, primäre, sekundäre und tertiäre aliphatische und aromatische Amine sowie heterocyclische Basen. Beispielhaft seinen Natrium-, Kalium- und Calziumhydroxid, Calziumoxid, Natrium- und Kaliumcarbonat, Natrium- und Kaliumhydrogencarbonat, Äthylamin, Methyl-äthylamin, Triäthylamin, Hydroxyäthylamin, Anilin, Dimethylanilin, Pyridin und Piperidin genannt. Bei Verwendung der silylierten Ausgangsstoffe sollten jedoch die oben angegebenen Einschränkungen bezüglich der Art der Basen beachtet werden.

Als Puffersysteme können alle üblichen Puffermischungen verwendet werden, z. B. Phosphatpuffer, Citratpuffer und Tris-(hydroxymethyl)-amino-methan-Puffer.

Die Reaktionstemperaturen können in einem größeren Bereich variiert werden. Im allgemeinen arbeitet man zwischen − 20 und + 50 °C, vorzugsweise zwischen 0 und + 20 °C.

Die Reaktionspartner der allgemeinen Formeln II und III können von vornherein in äquimolaren Mengen miteinander zur Reaktion gebracht werden. Es kann aber in einzelnen Fällen durchaus zweckmäßig sein, einen der beiden Reaktionspartner im Überschuß zu verwenden, um sich damit die Reinigung des Endproduktes zu erleichtern oder um die Ausbeute zur steigern.

2. Durch Umsetzung von Ureidocarbonsäuren der allgemeinen Formel IV,

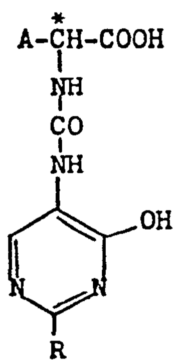

(IV)

in der A und R die oben angegebenen Bedeutungen besitzen, ihren Salzen oder reaktiven Derivaten, mit 7-Amino-cephalosporansäurederivaten der allgemeinen Formel V,

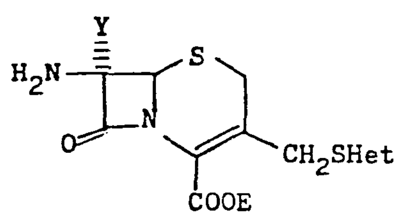

(V)

in der E, Y und Het wie oben definiert sind.

Als reaktive Derivate der Ureidocarbonsäuren der allgemeinen Formel IV kommen beispielsweise deren Säureanhydride wie z. B. die, die sich von Chlorameisensäureestern, z. B. Chlorameisensäureäthyl- oder isobutylester, ableiten, oder deren reaktive Ester, wie die p-Nitrophenylester oder der N-Hydroxysuccinimidester, oder deren reaktive Amide, wie das N-Carbonylimidazol, aber auch deren Säurehalogenide wie das entsprechende Säurechlorid oder deren Säureazide in Frage. Prinzipiell können jedoch alle Verknüpfungsmethoden, wie sie aus der β-Lactamchemie bekannt sind, verwendet werden.

Die 7-Aminocephalosporansäurederivate werden vorteilhafterweise in Form eines in vitro oder in vivo leicht spaltbaren Derivates eingesetzt. Hierfür kommen z. B. die Verbindungen der allgemeinen Formel V in Frage, bei denen E die eingangs gegebene Bedeutung mit Ausnahme der eines Wasserstoffatoms besitzt ; besonders bevorzugte Derivate sind der Diphenylmethylester, der t-Butylester, der Trimethylsilylester oder das N,O-Bis-trimethylsilylderivat.

Beispielsweise werden die Ureidocarbonsäure, ihre Salze oder ihre reaktiven Derivate mit den 7-Aminocephalosporansäurederivaten in einem Lösungsmittel bei Temperaturen zwischen − 40 °C und + 40 °C, gegebenenfalls in Gegenwart einer Base, umgesetzt. Wird z. B. ein Anhydrid der Ureidocarbonsäure, beispielsweise das Anhydrid mit dem Äthylchloroformiat, eingesetzt, so wird die Reaktion unter Kühlung, beispielsweise bei − 10 °C bis + 10 °C in Gegenwart eines tertiären Amins, wie Triäthylamin, oder N,N-Dimethylanilin, in einem Lösungsmittel, wie Aceton, Tetrahydrofuran, Dimethylformamid, Chloroform, Dichlormethan, Hexametapol oder in einem Gemisch dieser Lösungsmittel, durchgeführt. Setzt man beispielsweise einen N-Hydroxysuccinimidester der Ureidocarbonsäure mit Derivaten der allgemeinen Formel V um, so wird die Reaktion vorzugsweise bei 0 bis 20 °C in Anwesenheit einer Base, wie z. B. Triäthylamin, in einem Lösungsmittel wie Dimethylformamid, Dichlormethan, Dioxan oder in einem Gemisch solcher Lösungsmittel durchgeführt.

Die Umsetzung einer Ureidocarbonsäure der allgemeinen Formel IV selbst oder ihrer Salze mit Verbindungen der allgemeinen Formel V erfolgt vorteilhafterweise in Gegenwart eines Kondensationsmittels, z. B. in Gegenwart von N,N'-Dicyclohexylcarbodiimid.

3. Die Verbindungen der allgemeinen Formel I, in der E für Wasserstoff steht, können durch Umsetzung einer Verbindung der allgemeinen Formel VI,

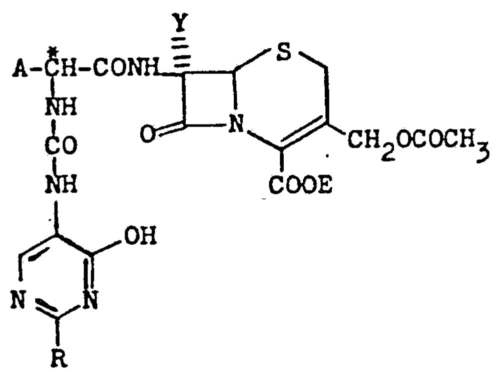

(VI)

in der A, R und Y die oben angegebenen Bedeutungen haben und E Wasserstoff bedeutet mit einer Verbindung der allgemeinen Formel VII,

Het—S—M (VII)

in der Het die oben angegebenen Bedeutungen hat und M für ein Wasserstoffatom oder ein Alkalimetall oder ein Erdalkalimetall steht, hergestellt werden. Dazu wird z. B. eine Verbindung der Formel VI mit beispielsweise 5-Vinyl-2-mercapto-1,2,3,4-tetrazol in einem Lösungsmittel, z. B. Wasser, Methanol, Äthanol, Aceton, Methyläthylketon, Tetrahydrofuran, Acetonitril, Essigsäureäthylester, Dimethoxyäthan, Dimethylformamid, Dimethylsulfoxid, Chloroform oder einem Gemisch dieser Lösungsmittel umgesetzt. Vorzugsweise wird ein stark polares Lösungsmittel, wie Wasser verwendet : In diesem Fall wird der pH-Wert der Reaktionslösung vorteilhafterweise auf 2-10 und insbesondere auf 4-8 gehalten. Der gewünschte pH-Wert kann durch Zugabe einer Pufferlösung, wie Natriumphosphat, eingestellt werden. Die Reaktionsbedingungen unterliegen keinen besonderen Beschränkungen. Normalerweise wird die Umsetzung bei einer Temperatur im Bereich von 0° bis 100 °C während einer Zeitdauer von einigen Stunden durchgeführt.

4. Eine Verbindung der allgemeinen Formel I, in der Y die Methoxygruppe darstellt, kann dadurch erhalten werden, daß man eine Verbindung der allgemeinen Formel I, in der Y ein Wasserstoffatom bedeutet, in Anwesenheit von Methanol mit einem Alkalimetall-Methylat der allgemeinen Formel $M^+OCH_3^-$, worin $M^+$ ein Alkalimetall bedeutet, und dann mit einem Halogenierungsmittel umsetzt. Dazu wird ein Cephalosporin der allgemeinen Formel I, in der Y ein Wasserstoffatom darstellt, in einem inerten Lösungsmittel, z. B. Tetrahydrofuran, Dioxan, Äthylengylcoldimethyläther, Methylenchlorid, Chloroform,

Dimethylformamid, Methanol oder dgl. oder in einem Gemisch von zweien dieser Lösungsmittel aufgelöst oder suspendiert. Zu der erhaltenen Lösung oder Suspension gibt man ein Alkalimetallmethylat zusammen mit Methanol. Das erhaltene Gemisch wird zur Reaktion gebracht und das Reaktionsgemisch wird dann mit einem Halogenierungsmittel umgesetzt. Bei dieser Reaktion verwendet man Methanol im Überschuß und die Menge des Alkalimetallmethylats beträgt vorzugsweise 2 bis 6 Äquivalente pro Äquivalent des verwendeten Cephalosporins. Der Ausdruck « im Überschuß » bedeutet eine Menge von mehr als einem Äquivalent pro Äquivalent des Cephalosporins. Alle Reaktionen werden bei – 120 bis – 10 °C und vorzugsweise – 100 °C bis – 50 °C durchgeführt. Eine Reaktionszeit von 5 bis 30 Min. reicht aus. Die Reaktion wird durch Ansäuern des Reaktionssystems abgebrochen.

Das bei diesem Verfahren eingesetzte Halogenierungsmittel ist allgemein als Quelle für positive Halogenatome, z. B. $Cl^+$ oder $Br^+$, $J^+$ bekannt. Beispiele solcher Halogenierungsmittel sind Halogen, wie Chlor, Brom usw. ; N-Halogenimide, wie N-Chlorsuccinimid, N-Bromsuccinimid und dgl. ; N-Halogenamide wie N-Chloracetamid, N-Bromacetamid, usw. ; N-Halogensulfonamide, wie N-Chlorbenzolsulfonamid, N-Chlor-p-toluolsulfonamid usw. ; 1-Halogenbenzotriazole ; 1-Halogentriazine, organische Hypohalogenite, wie tert.-Butylhypochlorit, tert.-Butylhypojodit usw. ; Halogenhydantoine, wie N,N-Dibromhydantoin, usw. Unter diesen Halogenierungsmitteln ist tert.-Butylhypochlorit bevorzugt. Das Halogenierungsmittel wird in einer Menge eingesetzt, welche ausreicht, eine zur Menge des Cephalosporins der allgemeinen Formel (VI) äquivalente Menge positives Halogen abzugeben.

Geeignete Säuren für das Abbrechen der Reaktion sind solche, welche bei Zusatz zum kaltern Reaktionsgemisch nicht zu einer Verfestigung des Reaktionsgemisches oder zum Gefrieren des Reaktionsgemisches zu einer schweren viskosen Masse führen. Beispiele geeigneter Säuren sind 98 %-ige Ameisensäure, Eisessig, Trichloressigsäure und Methansulfonsäure. Nach dem Abbrechen der Reaktion wird das überschüssige Halogenierungsmittel durch Behandlung mit einem Reduktionsmittel, z. B. Trialkylphosphit, Natriumthiosulfat oder dgl. entfernt.

Die nach den Verfahren 1, 2 und 4 hergestellten erfindungsgemäßen Verbindungen, in denen E für eine in vitro leicht abspaltbare Schutzgruppe steht, können nach bekannten Methoden der Cephalosporinchemie in die freien Carbonsäuren der allgemeinen Formel I, in der E ein Wasserstoffatom ist, überführt werden. So kann beispielsweise die Trimethylsilylgruppe leicht durch wäßrige Hydrolyse entfernt werden, die Benzhydrylgruppe z. B. durch hydrolytische Spaltung mit Trifluoressigsäure. Diese Eliminierung der Schutzgruppen sind literaturbekannt.

Ebenso können die Cephalosporinantibiotika der allgemeinen Formel I, in der E ein Wasserstoffatom bedeutet, in die Acyloxyalkylester, worin E z. B. einen Pivaloyloxymethylrest

$$-CH_2-O\underset{\underset{O}{\|}}{C}-C(CH_3)_3$$

darstellt, überführt werden, indem man ein Alkalisalz der Cephalosporincarbonsäure, beispielsweise ein Natrium- oder Kaliumsalz, mit einem Pivaloyloxymethylhalogenid der Formel

$$Hal-CH_2-O-\underset{\underset{O}{\|}}{C}-C(CH_3)_3,$$

worin Hal für Chlor, Brom oder Jod steht, umsetzt. Weitere geeignete Acyloxyalkylhalogenide sind z. B. Chlormethylacetat, Brommethylpropionat oder 1-Bromäthylacetat.

Die Herstellung des Acyloxyalkylesters der Formel I wird vorgenommen, indem man das jeweilige Alkalisalz der Stammsäure in einem inerten Lösungsmittel mit einem leichten molaren Überschuß des Jod-, Brom- oder Chloralkylesters, wie Pivaloyloxymethyljodid, bei Raumtemperatur oder leicht erhöhter Temperatur bis zu etwa 40 bis 45 °C umsetzt. Als Lösungsmittel Können beispielsweise Aceton, Tetrahydrofuran, Dioxan, Dimethylformamid oder Methylenchlorid verwendet werden.

Die Indanylester der Formel I, worin E für einen Rest der Formel

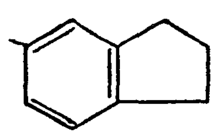

steht, können hergestellt werden, indem man 5-Indanol in einem inerten Lösungsmittel, wie Dioxan oder Tetrahydrofuran, mit der freien Säureform einer Verbindung der allgemeinen Formel I, worin E für Wasserstoff steht, in Gegenwart eines Kondensationsmittels, wie eines Diimids, beispielsweise Dicyclohexylcarbodiimid, umsetzt. Die Umsetzung wird unter Rühren bei einer Temperatur von etwa 20 bis 35 °C über eine Zeitspanne von etwa 6 bis 8 Stunden vorgenommen. Zur Isolierung des Indanylesters wird das Reaktionsgemisch erst mit Wasser verdünnt, worauf man den unlöslichen Dicyclohexylharnstoff vom Reaktionsgemisch abfiltriert. Der Ester wird dann aus dem Filtrat extrahiert.

Die Indanylester lassen sich ferner auch herstellen, indem man ein aus einer Cephalosporansäure der allgemeinen Formel I und Essigsäure gebildetes Anhydrid mit 5-Indanol umsetzt.

Die Phthalidylester der Formel I, worin $R_3$ für die Phthalidylgruppe der Formel

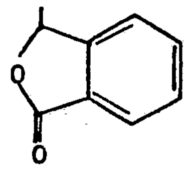

steht, können hergestellt werden, indem man Bromphthalid der Formel

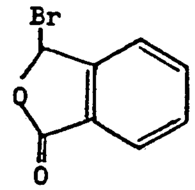

mit einem Salz einer Cephalosporansäure der allgemeinen Formel I umsetzt. Die Veresterung kann vorgenommen werden, indem man ein Gemisch aus äquimolaren Mengen des Cephalosporinsalzes, wie des Natrium- oder Kaliumsalzes, und Bromphthalid in Dimethylformamid, Dimethylacetamid, Dioxan, Tetrahydrofuran oder Gemischen hiervon langsam erwärmt.

Die Aufarbeitung der nach den genannten Verfahren erhaltenen Reaktionsgemische nach erfolgter Umsetzung wird nach den bei β-Lactam-Antibiotica gebräuchlichen Methoden vorgenommen ; dasselbe gilt für die Isolierung und Reinigung der Endprodukte, beispielsweise für die Freisetzung der Säure in andere Salze mittels anorganischer oder organischer Basen. Für die Herstellung der Kalium- oder Natriumsalze bewährt sich besonders die Fällung dieser Salze aus einer alkoholisch-ätherischen Lösung der freien Säure durch die Zugabe von Kalium- oder Natrium-2-äthylhexanoat oder die Umsetzung der freien Säure mit der entsprechenden Menge Natriumbicarbonat unter pH-Kontrolle und anschließender Gefriertrocknung.

Typische Ausgangsprodukte der allgemeinen Formeln II und V, bei denen A Phenyl, substituiertes Phenyl oder Thienyl bedeutet und Het z. B. für 1-Methyl-1H-tetrazol-5-yl steht, sind literaturbekannt, vgl. z. B. EP-OS 100 und DT-OS 2 934 682 und DT-OS 2 936 434. Beispielsweise erhält man solche 7-Aminocephalosporansäuresystemen, wenn man 7-Aminocephalosporansäure mit dem entsprechenden Mercaptoheterocyclus in bekannter Weise umsetzt.

Die Ausgangsstoffe der allgemeinen Formel III können beispielsweise durch Umsetzung der entsprechenden 5-Aminopyrimidine der allgemeinen Formel VIII,

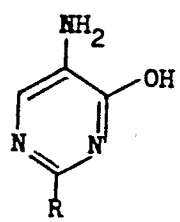

(VIII)

in der R wie oben definiert ist, mit Phosgen gewonnen werden. Diese Umsetzung erfolgt vorzugsweise in einem nicht hydroxylgruppen-enthaltenden Lösungsmittel, wie Tetrahydrofuran, Methylenchlorid, Chloroform, Dimethoxyäthan oder Hexametapol bei Temperaturen zwischen $-40°$ und $+60°$, vorzugsweise zwischen $-10°$ und $+20$ °C. Dabei empfiehlt es sich, den entstehenden Chlorwasserstoff durch äquimolare Mengen einer inerten organischen Base, wie Triäthylamin oder Pyridin, zu binden. Als Lösungsmittel kann auch Pyridin im Überschuß verwendet werden. Sollten die betreffenden Aminopyrimidine der allgemeinen Formel VIII in einem der erwähnten, Lösungsmittel schwer löslich sein, kann die Phosgenierung auch in heterogener Phase durchgeführt werden. Des weiteren können in einer besonders bevorzugten Ausführungsform die Aminopyrimidine der allgemeinen Formel VIII durch Behandlung mit einem Silylierungsmittel, wie Hexamethyldisilazan, Trimethylchlorsilan/Triäthylamin, Trimethylsilyldiäthylamin oder N,O-Bis-trimethylsilylacetamid in ein im allgemeinen in den erwähnten Lösungsmitteln sehr leicht lösliches, einfach oder, entsprechend den vorhandenen austauschbaren Wasserstoffatomen, mehrfach silyliertes Aminopyrimidin überführt werden, das mit Phosgen dann zu den entsprechenden Verbindungen der allgemeinen Formel III reagiert, wobei bevorzugt ohne Basenzusatz gearbeitet wird. Je nach der Art des Lösungsmittels, der höhe der Temperatur, der Menge und Art einer

eventuell zugesetzten Base entsteht entweder überwiegend das entsprechende Isocyanat oder Carbaminsäurehalogenid oder ein Gemisch dieser beiden Verbindungen. Je nach den Bedingungen liegt das Isocyanat der allgemeinen Formel III auch teilweise oder ganz als ein den Isocyanaten isomeres Dihydrooxazolo-pyrimidin der allgemeinen Formel IIIa

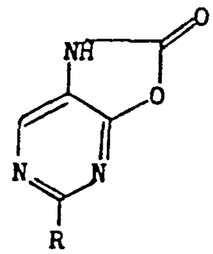

(IIIa)

oder ein je nach Art des Substituenten R ein oder mehrfach silyliertes Analoges vor.

Die durch Phosgenierung entstandenden Ausgangsprodukte der allgemeinen Formel III bzw. IIIa oder deren Gemische oder silylierte Analoge sind in den obenerwähnten Lösungsmitteln im allgemeinen gut löslich und können, nach Entfernung des überschüssigen Phosgens, ohne weitere Reinigung mit den entsprechenden Cephalosporinderivaten der allgemeinen Formel II direkt umgesetzt werden.

Es ist jedoch auch möglich, das Zwischenprodukt der allgemeinen Formel IIIa zu isolieren, es gegebenenfalls mit einem protischen Lösemittel, beispielsweise Wasser oder Methanol zu entsilylieren, es auf Grund seiner Löslichkeitseigenschaften zu reinigen und in der oben dargelegten Weise umzusetzen. Synthesen für 2-substituierte 5-Amino-4-hydroxy-pyrimidine der allgemeinen Formel VIII werden in den DT-OS 28 08 153 und 29 10 190 beschrieben.

Die Ureidocarbonsäuren der allgemeinen Formel IV lassen sich leicht durch Umsetzung der Pyrimidinderivate der allgemeinen Formel III mit Glycinderivaten der allgemeinen Formel IX,

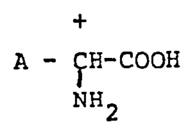

(IX)

in der A wie oben definiert ist, erhalten. Die Umsetzung erfolgt bei Temperaturen zwischen − 20° und 40 °C, vorzugsweise zwischen 0° und + 20 °C, in einem Lösungsmittel. Als Lösungsmittel können hierbei z. B. Gemische von Wasser und organischen Lösungsmitteln, die mit Wasser mischbar sind, verwendet werden, beispielsweise Aceton, Tetrahydrofuran, Dioxan, Acetonitril, Dimethylformamid, Äthanol, Dimethylsulfoxid. Gegebenenfalls wird die Verwendung eines halogenwasserstoffbindenden Mittels notwendig, als solche eignen sich beispielsweise Trialkylamine, wie Triäthylamin oder anorganische Basen, wie verdünnte Natronlauge. Diese Ureidocarbonsäure werden in der DT-OS 29 24 948 beschrieben.

Die Synthese der Ausgangsprodukte der allgemeinen Formel VI wird ebenfalls in der DT-OS 29 24 948 beschrieben.

Cephalosporinantibiotika werden in großem Umfang bei der Behandlung von Krankheiten verwendet, die durch pathogene Bakterien bei Mensch und Tier hervorgerufen werden, und sind insbesondere bei der Behandlung von Krankheiten verwendbar, die durch Bakterien hervorgerufen werden, welche gegenüber anderen Antibiotika, wie Penicillinverbindungen, resistent sind, sowie bei einer Behandlung von penicillinempfindlichen Patienten. In zahlreichen Fällen ist es erwünscht, ein Cephalosporinantibiotikum zu verwenden, das sowohl gegenüber grampositiven als auch gramnegativen Mikroorganismen eine Aktivität besitzt, und es wurden umfangreiche Untersuchungen auf die Entwicklung verschiedenartiger Typen von Cephalosporinantibiotika mit breitem Wirkungsspektrum gerichtet.

Bei diesen Untersuchungen hat es sich herausgestellt, daß es schwierig ist, Cephalosporinantibiotika zu finden, die neben einem breiten Wirkungsspektrum auch gegen verschiedenartige Stämme von Pseudomonas aeruginosa eine hohe Aktivität besitzen. Es wurde zwar versucht, entsprechend den Untersuchungen auf dem Penicillingebiet durch Acylierung von α-Aminobenzylcephalosporinen zu Pseudomonas-wirksamen Cephalosporinen zu gelangen, doch erwiesen sich derartige Verbindungen in der Regel als zu wenig aktiv. Es besteht daher weiter ein Bedürfnis, nach neuen Cephalosporinen zu suchen, die eine gesteigerte Wirksamkeit gegen verschiedenste Stämme von Pseudomonas aeruginosa neben einem breiten Wirkspektrum besitzen.

Während, wie erwähnt, allgemein über Acylderivate von α-Aminobenzylderivaten intensiv geforscht wurde und noch wird, ist nur wenig über Derivate bekannt geworden, bei denen ein heterocyclisches System über eine Ureidobrücke (—NHCONH—) an das α-Benzylkohlenstoffatom von α-Aminobenzylcephalosporinen geknüpft ist. Lediglich in der DT-OS 2 710 979 und DT-OS 2 650 826 werden Cephalosporine beschrieben, bei denen Pyridine bzw. kondensierte Pyridine in der genannten Weise mit dem Cephalosporingerüst verknüpft sind.

Diese Verbindungen weisen jedoch eine unbefriedigende Pseudomonaswirkung auf.

Im Gegensatz dazu wurde gefunden, daß einige der erfindungsgemäßen Verbindung hinsichtlich

ihrer antibiotischen Aktivität ein breites Wirkungsspektrum zusammen mit einer ungewöhnlich guten Wirksamkeit gegen Pseudomonas-Stämmen zeigen. Die hohe Aktivität erstreckt sich auch auf zahlreiche β-Lactamase bildende, gramnegative Stämme, da diese Verbindungen eine hohe Stabilität gegenüber β-Lactamasen, die von einer Reihe gramnegativer Organismen gebildet werden, besitzen. Außerdem sind die erfindungsgemäßen Wirkstoffe sehr gut verträglich, sie können daher zur Prophylaxe und Chemotherapie von lokalen und systemischen Infektionen in der Human- und Tiermedizin verwendet werden.

Als Krankheiten, die durch die erfindungsgemäßen Verbindungen verhindert bzw. geheilt werden können, seien beispielsweise solche der Atmungswege, des Rachenraumes und der Harnwege genannt ; die Verbindungen wirken insbesondere gegen Pharyngitis, Pneumonie, Peritonitis, Pyelonephritis, Otitis, Cystitis, Endocarditis, Bronchitis, Arthritis und allgemeine systemische Infektionen. Weiter können diese Verbindungen als Stoffe zur Konservierung von anorganischen oder organischen Materialien verwendet werden, besonders von organischen Materialien, wie Polymeren, Schmiermittel, Farben, Fasern, Leder, Papier und Holz sowie von Lebensmitteln.

Dieses wird, wie bereits dargelegt, dadurch ermöglicht, daß die Verbindungen der allgemeinen Formel I sowohl in vitro als auch in vivo schädliche Mikroorganismen, insbesondere gegen grampositive und gramnegative Bakterien und bakterienähnliche Mikroorganismen, sehr stark wirken, wobei sie sich besonders durch ein breites Wirkungsspektrum auszeichnen.

Mit diesen Cephalosporinderivaten können beispielsweise lokale und/oder systemische Erkrankungen behandelt und/oder verhindert werden, die durch die folgenden Erreger oder durch Mischungen der folgenden Erreger verursacht werden :

Micrococcaceae, wie Staphylokokken ;
Lactobacteriaceae, wie Streptokokken ;
Neisseriaceae, wie Neisserien ;
Corynebacteriaceae, wie Corynebakterien ;
Enterobacteriaceae, wie Escherichiae-Bakterien der Coli-Gruppe ;
Klebsiella-Bakterien, z. B. K. pneumoniae ;
Proteae-Bakterien der Proteus-Gruppe z. B. Proteus vulgaris ;
Salmonella-Bakterien, z. B. S. thyphimurium ;
Shigella-Bakterien, z. B. Shigella dysenteriae ;
Pseudomonas-Bakterien, z. B. Pseudomonas aeruginosa ;
Aeromonas-Bakterien, z. B. Aeromonas lique faciens ;
Spirillaceae, wie Vibrio-Bakterien, z. B. Vibrio cholerae ;
Parvobacteriaceae oder Brucellaceae, wie Pasteurella-Bakterien ;
Brucella-Bakterien, z. B. Brucella abortus ;
Haemophilus-Bakterien, z. B. Haemophilus influenzae ;
Bordetella-Bakterien, z. B. Bordetella pertussis ;
Moraxella-Bakterien, z. B. Moraxella lacunata ;
Bacteroidaceae, wie Bacteroides-Bakterien ;
Fusiforme-Bakterien, z. B. Fusobacterium fusiforme ;
Sphaerophorus-Bakterien, z. B. Sphaerophorus necrophorus ;
Bacillaceae, wie aerobe Sporenbildner, z. B. Bacillus anthracis ;
Anaerobe Sporenbildner-Chlostridien, z. B. Chlostridium perfringens ;
Spirochaetaceae, wie Borrelia-Bakterien ;
Treponema-Bakterien, z. B. Treponema pallidum ;
Leptospira-Bakterien, wie Leptospira interrogans.

Die obige Aufzählung von Erregern ist lediglich beispielhaft und keineswegs beschränkend aufzufassen.

In der folgenden Tabelle 1 werden einige typische, besonders gut wirksame Verbindungen entsprechend der vorliegenden Erfindung aufgezählt.

Tabelle 1

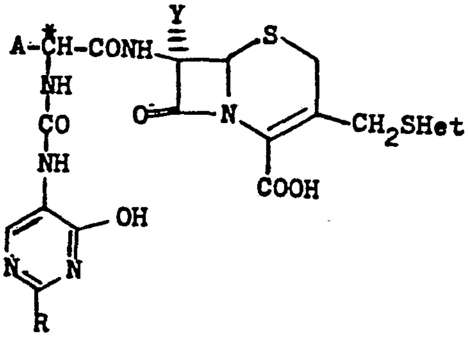

(Fortsetzung)

| | A | R | Y | Het |
|---|---|---|---|---|
| 1) | 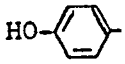 | 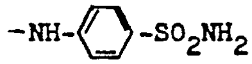 | H | 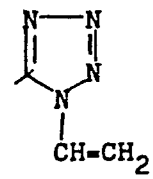 |
| 2) | 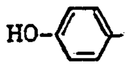 | 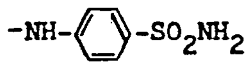 | H | 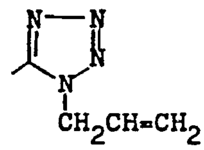 |
| 3) | 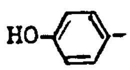 | 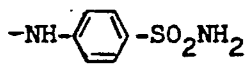 | H | 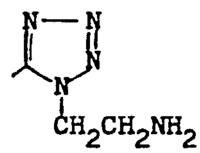 |
| 4) | 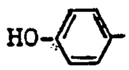 | 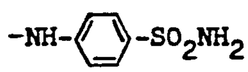 | H | 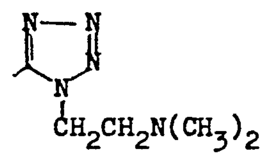 |
| 5) | 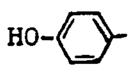 | 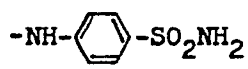 | H | 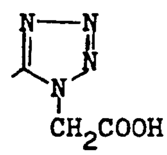 |
| 6) | 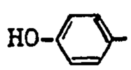 | 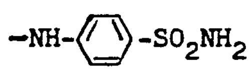 | H | 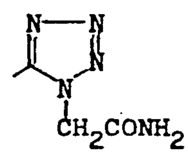 |
| 7) | 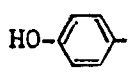 | 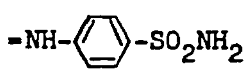 | H | 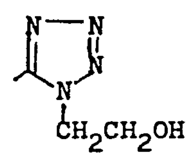 |
| 8) | 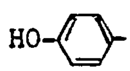 | 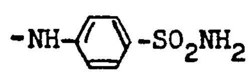 | H | 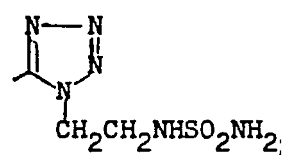 |
| 9) | 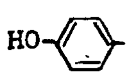 | 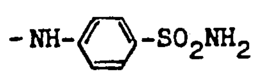 | H | 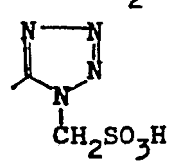 |
| 10) | 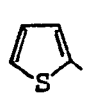 | 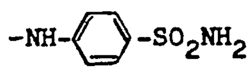 | H | 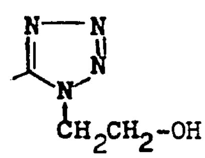 |
| 11) | 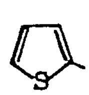 | 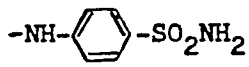 | $OCH_3$ | 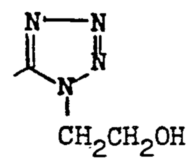 |

(Fortsetzung)

| | A | R | Y | Het |
|---|---|---|---|---|
| 12) | 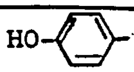 | 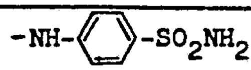 | $OCH_3$ | 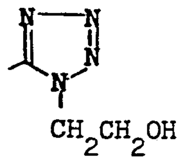 |
| 13) | 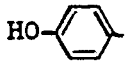 | 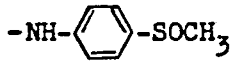 | H | 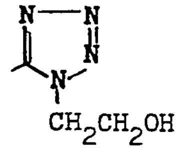 |
| 14) | 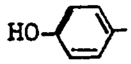 | 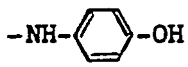 | H | 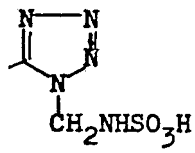 |
| 15) | 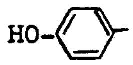 | 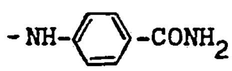 | H | 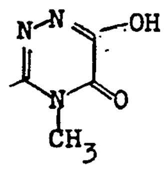 |
| 16) | 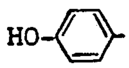 | 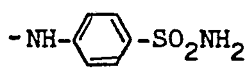 | H | 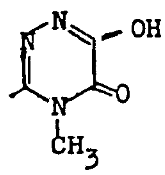 |
| 17) | 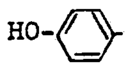 | 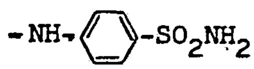 | $-OCH_3$ | 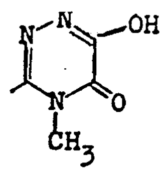 |
| 18) | 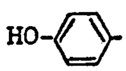 | 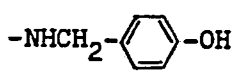 | H | 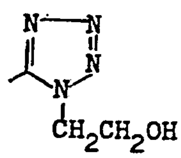 |
| 19) | 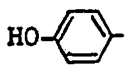 | 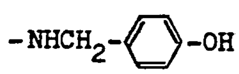 | H | 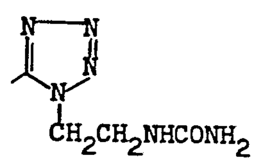 |
| 20) | 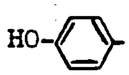 | 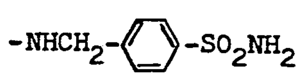 | H | 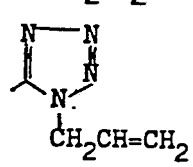 |
| 21) | 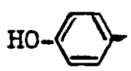 | 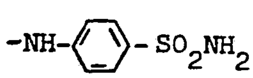 | H | 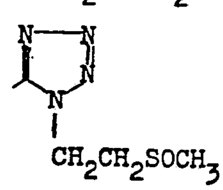 |
| 22) | 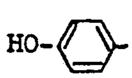 | 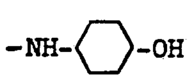 | H | 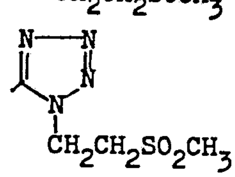 |

(Fortsetzung)

| | A | R | Y | Het |
|---|---|---|---|---|
| 23) | 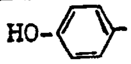 | $-NH(CH_2)_3OH$ | H | 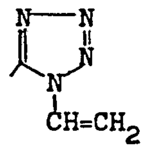 |
| 24) | 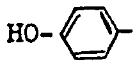 | $-NHCH_2CH_2SO_2NH_2$ | H | 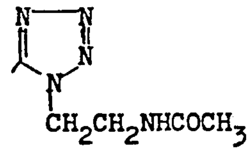 |
| 25) | 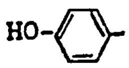 | $-NHC_3H_7$ | H | 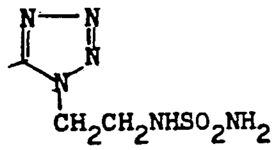 |
| 26) | 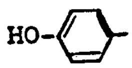 | 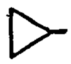 | H | 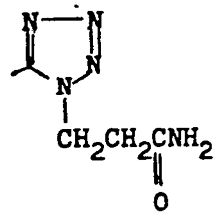 |
| 27) | 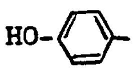 | 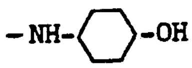 | H | 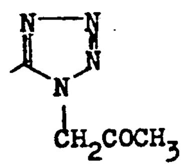 |
| 28) | 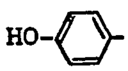 | 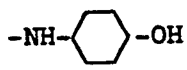 | H | 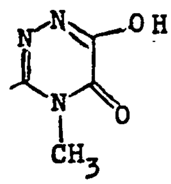 |
| 29) | 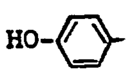 | 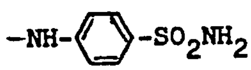 | H | 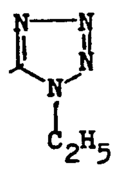 |
| 30) | 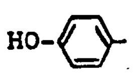 | 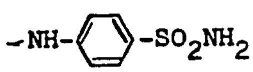 | H | 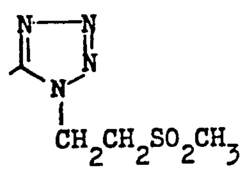 |
| 31) | 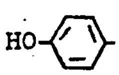 | 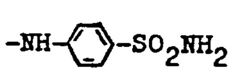 | H | 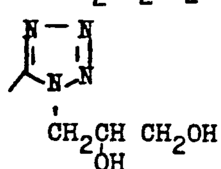 |
| 32) | 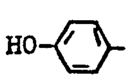 | 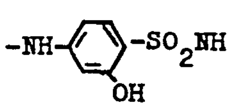 | H | 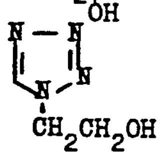 |

(Fortsetzung)

| | A | R | Y | Het |
|---|---|---|---|---|
| 33) | 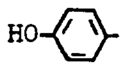 | 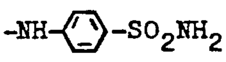 | H | 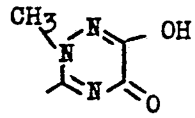 |
| 34) | 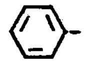 | 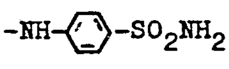 | $OCH_3$ | 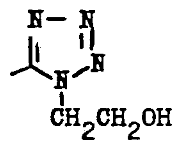 |
| 35) | 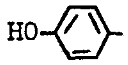 | 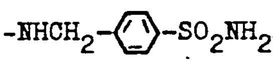 | H | 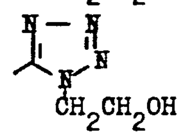 |
| 36) | 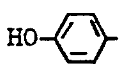 | 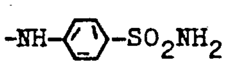 | H | 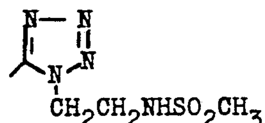 |
| 37) | 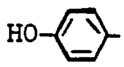 | 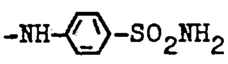 | H | 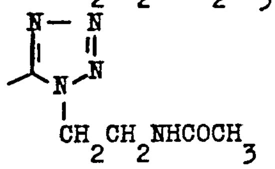 |

Die akute Toxizität wurde durch perorale und subcutane Applikation der Verbindungen der Tabelle 1 an weißen Laboratoriumsmäusen in steigenden Dosen bestimmt. Die $LD_{50}$ ist die Dosis, nach deren Applikation 50 % der Tiere innerhalb von 8 Tagen sterben. Sämtliche Substanzen zeigten bei oraler Gabe eine $LD_{50}$ von über 4 g/kg, bei subcutaner Gabe eine $LD_{50}$ von über 2 g/kg, d. h. bei 2 g/kg starben keine Tiere, sie sind damit für die Praxis untoxisch.

Die Wirksamkeit der erfindungsgemäßen Cephalosporine läßt sich durch folgende Untersuchungen beispielhaft demonstrieren :

1. In vitro-Versuche

Für die Untersuchungen wurde die Methode des Reihenverdünnungstestes im Mikrotitersystem angewandt. Die Prüfung der Substanzen auf Bakteriostase erfolgte in flüssigem Medium. Es wurde die Bakteriostasewirkung bei folgenden Konzentrationen untersucht :

$>$ 64, 64, 32, 16, 8, 4, 2, 1, 0,5, 0,25, 0,125, 0,06, 0,03, 0,015 µg/ml. Es wurde ein Nährboden folgender Zusammensetzung benutzt :

10 g Pepton, 8 g Fleischextrakt-Oxoid, 3 g Natriumchlorid, 2 g sek. Natriumphosphat werden mit dest. Wasser auf 100 ml aufgefüllt (pH 7,2-7,4). Lediglich bei der Testung gegen Streptokokken wurde 1 % Glucose hinzugefügt. Das Alter der Primärkulturen betrug etwa 20 Stunden. Die Einstellung der Keimsuspension erfolgte am Photometer (nach « Eppendorf ») (Reagenzglas-Durchmesser 14 mm, Filter 546 nm) anhand der Trübung einer Barium-Vergleichssuspension, die durch die Zugabe von 3,0 ml 1 %ige Barim-chloridlösung in 97 ml 1 %ige Schwefelsäure entstand. Nach der Einstellung wurden die Testkeime im Verhältnis 1 : 1 500 mit einer Kochsalzlösung weiter verdünnt.

16 mg der jeweiligen Substanz wurden in 10 ml-Meßkolben eingewogen und mit dem Lösungsmittel bis zur Marke aufgefüllt. Die weitere Verdünnungsreihe wurde mit destilliertem Wasser oder dem jeweiligen Lösungsmittel hergestellt.

Die Vertiefungen der Mikrotiterplatten wurden mit 0,2 ml Nährmedium, 0,01 ml der entsprechenden Substanzverdünnung und mit einem Tropfen Keimsuspension (0,01 ml) beschickt und 18 bis 20 Stunden bei 37 °C bebrütet. Eine Lösungsmittelkontrolle wurde stets mitgeführt.

Die Ablesung wurde makroskopisch vorgenommen, wobei die jeweilige Grenzkonzentration (= niedrigste noch bakteriostatisch wirksame Konzentration) ermittelt wurde.

Als Testorganismen wurden benützt :

Staphylococcus aureus SG 511, Escherichia coli ATCC 11 775, Pseudomonas aeruginosa Hamburgensis und Pseudomonas aeruginosa BC 19, Serratia marcescens ATCC 13 880, Klebsiella pneumoniae ATCC 10 031 und BC 6, Proteus mirabilis BC 17, Proteus rettgeri BC 7, Enterobacter cloaceae ATCC 13 047 und E. coli R + TEM (β-Lactamase-Träger).

In der folgenden Tabelle 2 sind die ermittelten minimalen Hemmkonzentrationen (MIC) für typische

Vertreter der erfindungsgemäßen Verbindungen aufgeführt (die Nummerierung entspricht derjenigen der Tabelle 1).

Als Vergleichsverbindungen werden zwei typische, sich im Handel befindliche Cephalosporine herangezogen.

Tabelle 2

MHK-Werte verschiedener Cephalosporine (in µg/ml)

| Verbindung | S.aureus SG 511 | E.coli ATCC 11775 | Pseud. aerug. Hbg. | Pseud. aerug. BC 19 | Klebs. pneum. ATCC 10031 | Klebs. pneum. BC 6 | Prot. mir. BC 17 | Prot. rettg. BC 7 | Eb. cloaceae | E.coli R+TEM | Serr. marcesc. ATCC 13880 |
|---|---|---|---|---|---|---|---|---|---|---|---|
| Cefuroxim | 1 | 8 | >100 | >100 | 2 | 4 | 0,5 | 2 | 32 | 4 | 8 |
| Cephazolin | 0,06 | 4 | >100 | >100 | 1 | 2 | 4 | >100 | >100 | 4 | >100 |
| 5 | 4 | 0,5 | 8 | 8 | 0,5 | 0,5 | 0,12 | 0,25 | 2 | 32 | 0,5 |
| 7 | 1 | 0,03 | 4 | 4 | 0,12 | 0,12 | 0,06 | 0,25 | 0,25 | 4 | 0,12 |
| 16 | 0,12 | 0,12 | 8 | 4 | 0,12 | 0,25 | 0,12 | 0,25 | 0,25 | 4 | 0,25 |
| 21 | 1 | 0,12 | 8 | 8 | 0,25 | 0,25 | 0,12 | 0,5 | - | 8 | 0,25 |
| 30 | 2 | 0,12 | 8 | 4 | 0,5 | 0,5 | 0,06 | 1 | 0,5 | 8 | 0,5 |
| 36 | 2 | 0,03 | 4 | 2 | 0,12 | 0,12 | 0,06 | 0,25 | 0,12 | 8 | 0,25 |
| 37 | 2 | 0,12 | 8 | 4 | 0,12 | 0,12 | 0,06 | 0,25 | 0,12 | 8 | 0,12 |

Wie aus der vorstehenden Tabelle ersichtlich wird, sind die genannten Verbindungen den Vergleichssubstanzen in ihrer Wirksamkeit gegenüber typischen gramnegativen Hospitalkeimen bei Erhalt der Wirkung gegen grampositive Keime deutlich überlegen. Hervorzuheben ist die gute Wirksamkeit gegen Pseudomonoasstämme.

Eine Reihe der erfindungsgemäßen Verbindungen wurde in vivo bei experimentellen Infektionen an Mäusen untersucht. Man verwendete als pathogene Bakterien E. coli ATCC 11 775 und Pseudomonas aeruginosa BC 19. Es wurde eine intraperitoneale Infektion mit 0,2 ml einer Bakteriensuspension (mit 5 % Mucin) gesetzt. Dies entspricht etwa $1{,}4 \times 10^6$ Keime E. coli bzw. $1{,}3 \times 10^6$ Keime Pseudomonas/Maus. Weibliche Mäuse vom Stamm NMRI wurden in Gruppen von jeweils 10 Tieren aufgeteilt, zwei Gruppen blieben unbehandelt, die restlichen Gruppen wurden mit verschiedenen Dosen der jeweiligen erfindungsgemäßen Cephalosporine subcutan zur Bestimmung der $ED_{50}$ (Dosis bei der 50 % der Tiere überleben) behandelt. Bei der E. coli-Infektion wurde einmal therapiert (1 h post-Infektionem). Bei der Pseudomonas-Infektion wurde dreimal behandelt (1, 3, 5 h post-Infektionem).

Der Beobachtungszeitraum betrug in beiden Fällen 7 Tage. Die Ergebnisse dieser Teste mit repräsentativen Vertretern der erfindungsgemäßen Cephalosporine sind in der Tabelle 3 dargestellt.

Tabelle 3

In vivo Aktivität bei Mäusen

a) E. coli-Infektion (s. c. Applikation):

| Verbindung | $ED_{50}$ (mg/kg) |
|---|---|
| 7 | ~ 0,3 |
| 16 | ~ 0,8 |
| 30 | ~ 0,6 |
| Cefuroxim | > 100 |

b) Pseudomonas (s. c. Applikation):

| Verbindung | $ED_{50}$ ($mg^+$/kg) |
|---|---|
| 7 | 6,3 |
| 16 | 10 - 20 |
| 30 | 10 - 20 |
| Cefuroxim | bei 200 mg/kg alle Tiere gestorben |

+ pro Dosis

Auch hierbei zeigt sich wieder eine signifikante Überlegenheit der erfindungsgemäßen Substanzen gegenüber der Vergleichssubstanz.

Es ist eine weitere Aufgabe der vorliegenden Erfindung, pharmazeutische Mittel zu schaffen, die bei der Behandlung infektiöser Krankheiten sowohl beim Menschen als auch beim Tier wertvoll sind.

Als bevorzugte pharmazeutische Zubereitungen seien Tabletten, Dragées, Kapseln, Granulate, Suppositorien, Lösungen, Suspensionen, Emulsionen, Salben, Gele, Cremes, Puder und Sprays genannt. Vorteilhafterweise wird der Wirkstoff in der Human- oder Tiermedizin oder ein Gemisch der verschiedenen Wirkstoffe der allgemeinen Formel I in einer Dosierung zwischen 5 und 500 vorzugsweise 10-200 mg/kg Körpergewicht je 24 Stunden verabreicht, gegebenenfalls in Form mehrerer Einzelgaben. Eine Einzelgabe enthält den oder die erfindungsgemäßen Wirkstoffe, vorzugsweise in Mengen von etwa 1 bis etwa 250, insbesondere 10 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Im Falle der Anwendung als Futterzusatzmittel können die neuen Verbindungen in den üblichen

Konzentrationen und Zubereitungen zusammen mit dem Futter bzw. mit Futterzubereitungen oder mit dem Tinkwasser gegeben werden. Dadurch kann eine Infektion durch gramnegative oder grampositive Bakterien verhindert, gebessert und/oder geheilt werden und ebenso eine Förderung des Wachstums und eine Verbesserung der Verwertung des Futters erreicht werden.

Die folgenden Beispiele sollen die Erfindung näher erläutern :

Beispiel 1

Natrium-7-{D-α-[3-(2-p-aminosulfonylanilino-4-hydroxy-5-pyrimidinyl)-ureido]-p-hydroxyphenylacetamido}-3-[(1-vinyltetrazol-5-yl)-thiomethyl]-ceph-3-em-4-carboxylat

2,37 g (0,005 Mol) D-α-[3-(2-p-Aminosulfonylanilino-4-hydroxy-5-pyrimidinyl)-ureido]-p-hydroxy-phenylessigsäure werden in 30 ml trockenem Dimethylformamid gelöst. Man fügt eine Lösung von 2,53 g (0,005 Mol) 7-Amino-3-[(1-vinyltetrazol-5-yl)-thiomethyl]-ceph-3-em-4-carbonsäure-diphenylmethylester in 30 ml trockenem Methylenchlorid hinzu. Zu dieser Lösung werden unter Eiskühlung 1,13 g Dicyclohexylcarbodiimid gegeben und es wird 2 Stunden bei 10 °C, und 6 Stunden bei Raumtemperatur gerührt. Ein Dünnschichtchromatogramm zeigt, daß die Ausgangsprodukte nahezu vollständig verschwunden sind. Man filtriert und engt dann das Filtrat im Vakuum zur Trockne ein, rührt zweimal mit jeweils 50 ml Methanol und einmal mit 100 ml Methylenchlorid gut aus. Das zurückbleibende Festprodukt wird abgesaugt und mit Äther gut gewaschen. Zur Entfernung geringfügiger Verunreinigungen, die im Dünnschichtchromatogramm (Methylenchlorid : Methanol 5 : 1) am Startfleck sitzen bleiben, wird über eine Kieselgelsäule chromatographiert. Ausbeute an Ester : 3,46 g (72 %).

Das so erhaltene Produkt wird in wenig Methylenchlorid suspendiert und unter Eiskühlung 30 Minuten mit 2 ml Anisol und 10 ml Trifluoressigsäure gerührt, wobei Lösung eintritt. Anschließend werden zweimal 50 ml Toluol hinzugefügt und im Vakuum jeweils zur Trockne eingeengt. Man versetzt mit Äther und saugt ab.

Zur Herstellung des Natriumsalzes löst man in wenig Dimethylformamid, gibt die berechnete Menge Natrium-äthylhexanoat in Methanol zu und versetzt mit Äther. Das ausgefällte Produkt wird abgesaugt, sorgfältig mit Äther gewaschen, und im Vakuum getrocknet.

Ausbeute an Natriumsalz (bezogen auf das eingesetzte Cephalosporinderivat) : 2,71 g (66 %).

IR-Spektrum : 1 760, 1 655, 1 615, 1 550 $cm^{-1}$ ;

NMR-Spektrum (DMSO + $CD_3OD$) Signale bei ppm : 3,45 (m, 2H), 4,35 (m, 2H), 4,85 (d, 1H), 5,40-5,90 (m, 4H), 6,6-7,0 (m, 2 + 1H), 7,15 (d, 2H), 7,7 (d, 2H), 8,0 (d, 2H), 8,37 (s, 1H).

Nach der in Beispiel 1 angegebenen Methode wurden die Cephalosporine der folgenden Tabelle synthetisiert.

(Siehe Tabelle Seite 18 f.)

Tabelle

| Beispiel | A | R | Y | Het | Ausbeute % | IR-Sp. ($cm^{-1}$) | NMR-Spektrum (DMSO + $CD_3OD$) Signale bei ppm: |
|---|---|---|---|---|---|---|---|
| 2 | 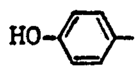 | 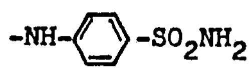 | H | 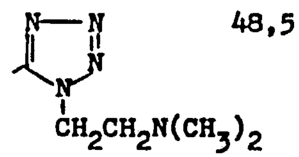 | 48,5 | 1760, 1650, 1610 | 2,25(s,6H), 2,75 (t,2H), 3,60 (m,2H), 4,30(m,4H), 4,85(d,1H), 5,40(s,1H), 5,55(d,1H), 6,75 (d,2H), 7,25(d,2H), 7,75 (d,2H), 8,0(d,2H), 8,38(s,1H). |
| 3 | 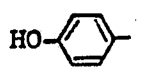 | 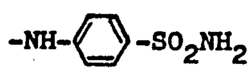 | H | 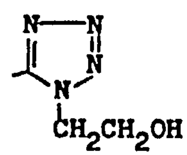 | 44 | 1760, 1660, 1610 | 3,6(m,2H), 3,80(m,2H), 4,35 (m,4H), 4,95(d,1H), 5,40(s, 1H), 5,70(d,1H), 6,75(d,2H), 7,25(d,2H), 7,70(d,2H), 8,0 (d,2H), 8,36(s,1H). |
| 4 | 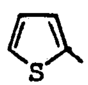 | 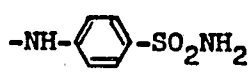 | H | 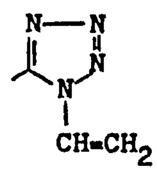 | 52 | 1760, 1650, 1600 | 3,45(m,2H), 4,35(m,2H), 4,95 (dd,1H), 5,50-5,90(m,4H), 6,7-7,2(m,3H), 7,35(m,1H), 7,7(d,2H), 8,0(d,2H), 8,35 (s,1H). |
| 5 | 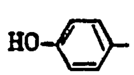 | 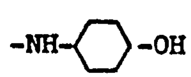 | H | 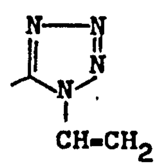 | 58 | 1760, 1650, 1600 | 1,75(m,8H), 3,4-3,8(m,1+1H+ 2H), 4,4(m,2H), 4,85(d,1H), 5,40-5,90(m,4H), 6,60-7.0 (m,3H), 7,25(d,2H), 7,70 (d,2H), 8,0(d,2H), 8,35(s,1H). |

Beispiel 6

Natrium-7-{D-α-[3-(2-p-aminosulfonylanilino-4-hydroxy-5-pyrimidinyl)-ureido]-p-hydroxyphenylacetamido}-3-[(1-allyltetrazol-5-yl)-thiomethyl]-ceph-3-em-4-carboxylat

Zu einer Lösung von 950 mg (0,002 Mol) der Ureidocarbonsäure, die in Beispiel 1 eingesetzt wurde, in 20 ml trockenem Dimethylformamid gibt man 0,2 g N-Methylmorpholin. Die Lösung wird auf − 15 °C abgekühlt und es wird bei dieser Temperatur eine Lösung von 0,22 g Chlorameisensäureäthylester in 5 ml Methylenchlorid zugetropft. Die erhaltene Mischung wird 45 Minuten bei dieser Temperatur gehalten. Anschließend tropft man bei − 15 °C eine Lösung von 1,04 g (0,002 Mol) 7-Amino-3-[(1-allyl-tetrazol-5-yl)-thiomethyl]-ceph-3-em-4-carbonsäure-diphenylmethylester in 20 ml trockenen Methylenchlorid zu. Man rührt 1 Stunde bei − 10 °C und läßt dann langsam auf Raumtemperatur kommen. Die Lösung wird im Vakuum zur Trockne eingeengt und, wie in Beispiel 1 beschrieben, weiter behandelt.

Die Abspaltung der Esterschutzgruppe erfolgt ebenfalls analog Beispiel 1.

Ausbeute an Natriumsalz : 900 mg (54 %) ;

IR-Spektrum : 1 760, 1 650, 1 610, 1 550 $cm^{-1}$ ;

NMR-Spektrum (DMSO + $CD_3OD$) Signale bei ppm : 3,60 (m, 2H), 4,25 (m, 2H), 4,80-5,60 (m, 6H), 5,70-6,40 (m, 2H), 6,75 (d, 2H), 7,25 (d, 2H), 7,70 (d, 2H), 8,0 (d, 2H), 8,36 (s, 1H).

Analog Beispiel 6 wurden folgende Cephalosporine synthetisiert :

(Siehe Tabelle Seite 20 f.)

Tabelle

| Beispiel | A | R | Y | Het | Ausbeute % | IR-Spektrum $cm^{-1}$ | NMR-Spektrum (DMSO + $CD_3OD$) Signale bei ppm: |
|---|---|---|---|---|---|---|---|
| 7 | 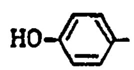 | 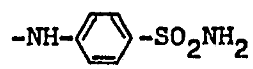 | H | 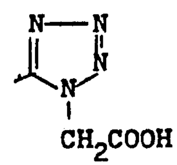 | 45 | 1760, 1650, 1610 | 3,6(m,2H), 4,3(m,2H), 5,0(m,2+1H), 5,55(s,1H), 5,70(d,1H), 6,75(d,2H), 7,30(d,2H), 7,85(m,4H), 8,24(s,1H). |
| 8 | 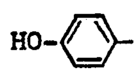 | 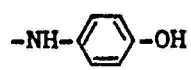 | H | 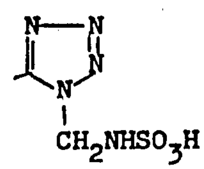 | 52 | 1760, 1660, 1605 | 3,55(m,2H), 4,35 (m,2H), 4,8(s,2H), 4,95(d,1H), 5,50(s,1H), 5,65(d,1H), 6,75(d,2H), 7,15(d,2H), 7,45(d,2H), 7,85(d,2H), 8,30(s,1H). |
| 9 | 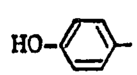 | 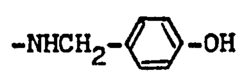 | H | 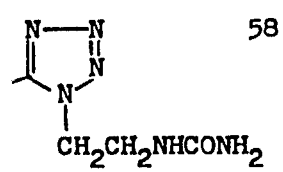 | 58 | 1760, 1660, 1600 | 3,0(m,2H), 3,50(m,2H), 4,4(m,6H), 4,90(d,1H), 5,55(d,1H), 5,70(d,1H), 6,85(d,2H), 7,15-7,6(m, 6H), 8,05(s,1H). |

Beispiel 10

7-{D-α-[3-(2-p-Aminosulfonylanilino-4-hydroxy-5-pyrimidinyl)-ureido]-p-hydroxyphenylacetamido}-3-[(1-hydroxysulfonylmethyltetrazol-5-yl)-thiomethyl]-ceph-3-em-4-carbonsäure

Aus 475 mg (0,001 Mol) der Ureidocarbonsäure, die in Beispiel 1 eingesetzt wurde, wird analog Beispiel 6 mit N-Methylmorpholin und Chlorameisensäureäthylester das aktivierte Anhydrid hergestellt. Andererseits gibt man zu einer Suspension von 410 mg (0,001 Mol) 7-Amino-3-[(1-hydroxysulfonylmethyl-tetrazol-5-yl)-thiomethyl]-ceph-3-em-4-carbonsäure in 20 ml wasserfreiem Acetonitril 600 mg N,O-Bistrimethylsilylacetamid, wobei eine Lösung erhalten wird. Diese Lösung wird auf – 15 °C abgekühlt und zu der oberen Lösung bei dieser Temperatur zugetropft. Danach wird die Mischung bei – 10 °C 1 Stunde und bei + 10 °C ebenfalls 1 Stunde lang gerührt. Nach dieser Zeit fügt man 2 ml Methanol zu und filtriert vom unlöslichen Material ab. Danach wird das Lösungsmittel im Vakuum entfernt. Der Rückstand wird in 40 ml Wasser aufgenommen und auf pH 7,0 gestellt. Bei diesem pH-Wert wird zweimal mit Essigester ausgeschüttelt. Die wäßrige Phase wird unter Eiskühlung mit verdünnter Salzsäure auf pH 2,9 gestellt, das ausgefallene Produkt wird abgesaugt, mit wenig Wasser gewaschen und im Vakuum getrocknet.

Ausbeute : 435 mg (59 %) ;

IR-Spektrum : 1 760, 1 660, 1 600 $cm^{-1}$ ;

NMR-Spektrum (DMSO + $CD_3OD$) Signale bei ppm : 3,60 (m, 2H), 4,25 (m, 2H), 5,0 (m, 3H), 5,55 (s, 1H), 5,70 (d, 1H), 6,75 (d, 2H), 7,25 (d, 2H), 7,85 (m, 4H), 8,37 (s, 1H).

Analog Beispiel 10 wurden folgende Cephalosporine synthetisiert :

(Siehe Tabelle Seite 22 ff.)

Tabelle

| Beispiel | A | R | Y | Het | Ausbeute % | IR-Spektrum $cm^{-1}$ | NMR-Spektrum (DMSO + $CD_3OD$) Signale bei ppm: |
|---|---|---|---|---|---|---|---|
| 11 | 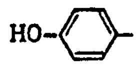 | 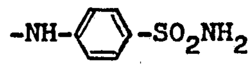 | H | 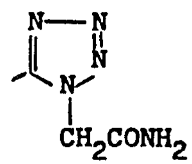 | 58,5 | 1760, 1660, 1615 | 3,60(m,2H), 4,35(m,2H), 5,0(m,2+1H), 5,50(s,1H), 5,65(d,1H), 6,75(d,2H), 7,25(d,2H), 7,7(d,2H), 8,0(d,2H), 8,30(s,1H). |
| 12 | 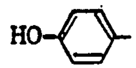 | 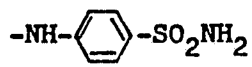 | H | 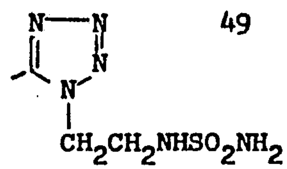 | 49 | 1760, 1650, 1610 | 2,95(m,2H), 3,50(m,2H), 4,4(m,4H), 4,95(d,1H), 5,55(s,1H), 5,70(d,1H), 6,85(d,2H), 7,25(d,2H), 7,70(d,2H), 8,0(d,2H), 8,35(s,1H). |
| 13 | 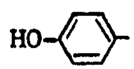 | $-NH-CH_2CH_2SO_2NH_2$ | H | 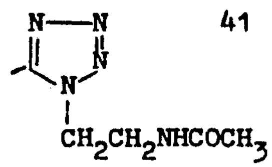 | 41 | 1760, 1660, 1600 | 1,85(s,3H), 3,0(m,2H), 3,4-3,8(m,4H), 4,45(m,2+2H), 4,90(d,1H), 5,55(s,1H), 5,70(d,1H), 6,75(d,2H), 7,25(d,2H), 8,05(s,1H), 3,0-3,65(m,8H). |
| 14 | 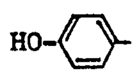 | 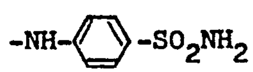 | H | 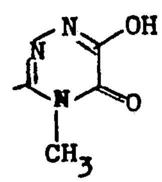 | 44 | 1760, 1665, 1605 | 3,40(s,3H), 3,55(m,2H), 4,25(m,2H), 4,90(d,1H), 5,55(s,1H), 5,70(d,1H), 6,85(d,2H), 7,25(d,2H), 7,70(d,2H), 8,05(d,2H), 8,36(s,1H). |

(Fortsetzung)

| Beispiel | A | R | Y | Het | Ausbeute % | IR-Spektrum $cm^{-1}$ | NMR-Spektrum (DMSO + $CD_3OD$) Signale bei ppm: |
|---|---|---|---|---|---|---|---|
| 15 | 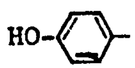 | 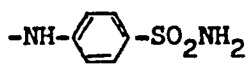 | H | 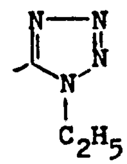 | 52 | 1760, 1670, 1650 | 1,0(t,3H), 3,6(m,2H), 4,4(m,4H), 4,90(d,1H), 5,50(s,1H), 5,65(d,1H), 6,75(d,2H), 7,25(d,2H), 7,70(d,2H), 8,0(d,2H), 8,35(s,1H). |
| 16 | 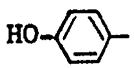 | 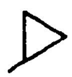 | H | 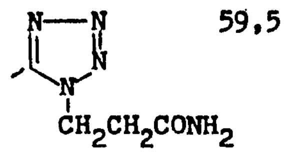 | 59,5 | 1760, 1660, 1605 | 1,20(m,4H), 1,95(m,1H), 3,0(m,2H), 3,55(m,2H), 4,35(m,4H), 4,95(d,1H), 5,55(s,1H), 5,70(d,1H), 6,75(d,2H), 7,25(d,2H), 8,40(s,1H). |
| 17 | 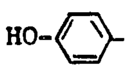 | 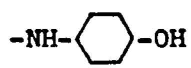 | H | 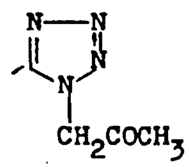 | 38 | 1760, 1660, 1610 | 1,75(m,8H), 2,20(s,3H), 3,50-3,8(m,4H), 4,0-4,4 (m,4H), 4,90(d,1H), 5,55 (s,1H), 5,70(d,1H), 6,75 (d,2H), 7,25(d,2H), 8,05 (s,1H). |
| 18 | 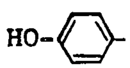 | $-NHC_3H_7$ | H | 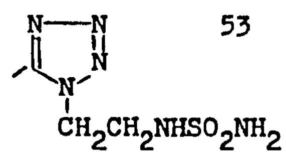 | 53 | 1760, 1670, 1600 | 1,0(t,3H), 1,6(q,2H), 3,0-3,6(m,6H), 4,35(m,4H), 4,95(d,1H), 5,55(s,1H), 5,70(d,1H), 6,75(d,2H), 7,25(d,2H), 8,10(s,1H). |

Beispiel 19

Natrium-7-{D-α-[3-(2-p-aminosulfonylanilino-4-hydroxy-5-pyrimidinyl)-ureido]-p-hydroxy-phenylacetamido}-3-[(1-(2'-aminoäthyl)-tetrazol-5-yl)-thiomethyl]-ceph-3-em-4-carboxylat

Die Herstellung erfolgte analog Beispiel 1, ausgehend von der dort eingesetzten Ureidocarbonsäure sowie einer äquimolekularen Menge 7-Amino-3-[(1-(2'-t-butoxycarbonylamino-äthyl)-tetrazol-5-yl)-thiomethyl]-ceph-3-em-4-carbonsäure-diphenylmethylester. Ausbeute nach Abspaltung der Schutzgruppen und Herstellung des Natriumsalzes : 43,5 % ;

IR-Spektrum : 1 760, 1 675, 1 600 $cm^{-1}$ ;

NMR-Spektrum (DMSO + $CD_3OD$) Signale bei ppm : 3,15 (m, 2H), 3,55 (m, 2H), 4,35 (m, 4H), 4,95 (d, 1H), 5,55 (s, 1H), 5,70 (d, 1H), 6,75 (d, 2H), 7,25 (d, 2H), 7,70 (d, 2H), 8,0 (d, 2H), 8,35 (s, 1H).

Beispiel 20

Natrium-7-β-{D-α-[3-(2-p-aminosulfonylanilino-4-hydroxy-5-pyrimidinyl)-ureido]-p-hydroxyphenylacetamido}-7α-methoxy-3-[(1-vinyl-tetrazol-5-yl)-thiomethyl]-ceph-3-em-4-carboxylat

In einem Gemisch von 40 ml wasserfreiem Methanol und 10 ml wasserfreiem Tetrahydrofuran werden 960 mg des in Beispiel 1 erhaltenen Diphenylmethylesters vorgelegt. Bei − 70 °C wird eine Lösung von Lithiummethoxid zugegeben, die 7 mMol enthält. Man rührt 5 Minuten und gibt zu der Lösung dann 0,18 ml tert.-Butylhypochlorit. Die Mischung wird 30 Minuten bei − 70 °C gerührt, worauf man 0,45 ml Essigsäure und 0,15 ml Triäthylphosphit zugibt. Danach läßt man auf Raumtemperatur kommen. Man dampft die Suspension im Vakuum zur Trockne ein. Das zurückbleibende Festprodukt wird zweimal mit 50 ml Methanol und einmal mit 450 ml Methylenchlorid ausgerührt und abgesaugt.

Zur Reinigung wird über eine Fertigsäule chromatographiert (Eluens Methylenchlorid : Methanol 6 : 1).

Ausbeute : nach Abspaltung der Schutzgruppe 37 % ;

IR-Spektrum : 1 760, 1 670, 1 600 $cm^{-1}$ ;

NMR-Spektrum (DMSO + $CD_3OD$) Signale bei ppm : 3,45 (s,3H), 3,55 (m,2H), 4,35 (m,2H), 5,0 (s,1H), 5,40-5,90 (m,3H), 6,6-7,0 (m,2+1H), 7,15 (d,2H), 7,7 (d,2H), 8,0 (d,2H), 8,35 (s,1H).

Beispiel 21

Natrium-7β-{D,L-α-[3-(2-p-aminosulfonylanilino-4-hydroxy-5-pyrimidinyl)-ureido]-2-thienylacetamido}-7α-methoxy-3-[1-(vinyltetrazol-5-yl)-thiomethyl-]-ceph-3-em-4-carboxylat

Die Synthese erfolgte analog Beispiel 20, ausgehend von dem Cephalosporindiphenylmethylester, der in Beispiel 4 als Zwischenprodukt anfiel.

Ausbeute : 29 % :

IR-Spektrum : 1 760, 1 660, 1 600 $cm^{-1}$ ;

Beispiel 22

Natrium-7-{D-α-[3-(4-hydroxy-2-p-hydroxybenzylamino-5-pyrimidinyl)-ureido]-p-hydroxy-phenylacetamido}-3-[(1-(2'-hydroxyäthyl)-tetrazol-5-yl)-thiomethyl]-ceph-3-em-4-carboxylat

Ausgehend von 2,18 g D-α-[3-(4-Hydroxy-2-p-hydroxybenzylamino-5-pyrimidinyl)-ureido]-p-hydroxy-phenylessigsäure (0,005 Mol) und 1,36 g 7-Aminocephalosporansäure werden analog der Herstellvorschrift des Beispiels 10 2,08 g (62 %) 7-{D-α-[3-(4-hydroxy-2-p-hydroxybenzylamino-5-pyrimidinyl)-ureido]-p-hydroxy-phenylacetamido}-3-acetoxymethyl-ceph-3-em-4-carbonsäure erhalten.

500 mg dieses Cephalosporins werden in 20 ml einer Phosphorsäurepufferlösung von pH 6,3 zusammen mit 200 g 1-(2'-Hydroxyäthyl)-5-mercapto-tetrazol 6 Stunden unter Stickstoff auf 70 °C erwärmt, wobei der pH-Wert zwischen 6 und 6,5 gehalten wird. Nach dieser Zeit wird die Reaktionsflüssigkeit abgekühlt, von etwas Unlöslichem abfiltriert und zweimal mit Essigsäureäthylester ausgeschüttelt. Anschließend wird unter Kühlen Salzsäure bis zu einem pH-Wert von 2,8 zugesetzt. Das ausgefallene Produkt wird abgesaugt, mit wenig Wasser gewaschen und getrocknet. Der Rückstand wird auf die übliche Weise in das Natriumsalz überführt.

Ausbeute : 64 % ;

IR-Spektrum : 1 760, 1 660, 1 600 $cm^{-1}$ ;

NMR-Spektrum : 3,0 (m,2H), 3,50 (2H), 4,1-4,5 (m,6H), 4,95 (d,1H), 5,50 (s,1H), 5,65 (d,1H), 6,75 (d,2H), 7,25 (d,2H), 7,70 (d,2H), 8,0 (d,2H), 8,36 (s,1H).

Beispiel 23

Natrium-7-{D-α-[3-(4-hydroxy-2-(4'-hydroxycyclohexylamino)-5-pyrimidinyl)-ureido]-3-[(1-(2'-methylsulfonyläthyl)-tetrazol-5-yl)-thiomethyl]-ceph-3-em-4-carboxylat

Dieses Cephalosporin wird analog Beispiel 22 synthetisiert, wobei man von 7-{D-α-[3-(4-Hydroxy-2-(4'-hydroxycyclohexylamino)-5-pyrimidinyl)-ureido]-3-acetoxymethyl-ceph-3-em-4-carbonsäure und 1-(2'-Methylsulfonyläthyl)-5-mercapto-tetrazol ausgeht.

Ausbeute : 48,5 % (der Theorie) ;

IR-Spektrum : 1 760, 1 600 $cm^{-1}$ ;

NMR-Spektrum (DMSO + $CD_3OD$) Signale bei ppm : 1,8 (m,8H), 2,85 (s,3H), 3,3-4,0 (m,6H), 4,35 (m,4H), 4,95 (d,1H), 5,55 (s,1H), 5,70 (d,1H), 6,75 (d,2H), 7,25 (d,2H), 8,05 (s,1H).

Beispiel 24

Natrium-7-{D-α-[3-(4-hydroxy-2-(4'-hydroxycyclohexylamino)-5-pyrimidinyl)-ureido]-3-[(1-methyl-5,6-dioxo-1,3,4-triazin-2-yl)-thiomethyl]-ceph-3-em-4-carboxylat

Die Synthese erfolgte analog Beispiel 23, ausgehend von dem Cephalosporinderivat, das dort eingesetzt wurde sowie 4-Methyl-2-mercapto-5,6-dioxo-1,3,4-triazin.

Ausbeute : 66,5 % (der Theorie) ;

IR-Spektrum : 1 760, 1 670, 1 600 $cm^{-1}$ ;

NMR-Spektrum (DMSO + $CD_3OD$) Signale bei ppm : 1,8 (m,8H), 3,5 (m,2H+s,3H), 4,35 (m,2H), 4,90 (d,1H), 5,50 (s,1H), 5,65 (d,1H), 6,75 (d,2H), 7,25 (d,2H), 8,05 (s,1H).

Beispiel 25

Natrium-7-{D-α-[3-(2-p-aminosulfonylanilino-4-hydroxy-5-pyrimidinyl)-ureido]-p-hydroxyphenylacetamido}-3-[(1-(2'-methylsulfonyläthylamino)-tetrazol-5-yl)-thiomethyl]-ceph-3-em-4-carboxylat

Die Synthese erfolgte analog Beispiel 1, ausgehend von der dort angegebenen Ureidocarbonsäure sowie dem entsprechenden Cephalosporin-benzhydrylester.

Ausbeute : 49,5 % ;

IR-Spektrum : 1 765, 1 660, 1 590 $cm^{-1}$ ;

NMR-Spektrum (DMSO + $CD_3OD$) Signale bei ppm : 2,9 (s,3H), 3,6 (m,4H), 4,4 (m,4H), 5,0 (d,1H), 5,4 (s,1H), 5,75 (d,1H), 6,8 (d,2H), 7,3 (d,2H), 7,8 (s,1H), 8,32 (s,1H),

analog wurden hergestellt :

(Siehe Tabelle Seite 26 f.)

Tabelle

| Beispiel | A | R | Y | Het | IR-Spektrum $cm^{-1}$ | NMR-Spektrum (DMSO + $CD_3OD$) Signale bei ppm: |
|---|---|---|---|---|---|---|
| 26 | 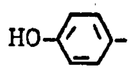 | 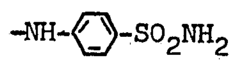 | H | 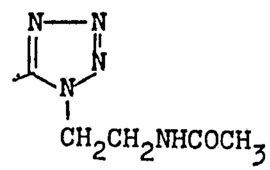 | 1760 1655 | 1,8(s,3H), 3,5-3,7(m,4H), 4,5(m,2H), 4,9(d,1H), 5,4 (s,1H), 5,60(d,1H), 6,7 (d,2H), 7,2(d,2H), 7,8 (q,4H), 8,35(s,1H), |
| 27 | 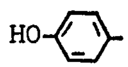 | 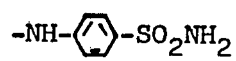 | H | 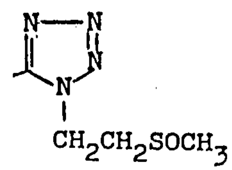 | 1760 1660 1600 1150 | 2,6(s,3H), 3,2-3,6(m,4H), 4,3(m,2H), 4,65(m,2H), 4,9 (d,1H), 5,4(d,1H), 5,55 (d,1H), 6,65(d,2H), 7,2 (d,2H), 7,65(d,2H), 7,9 (d,2H), 8,30(s,1H) |
| 28 | 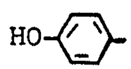 | 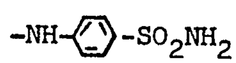 | H | 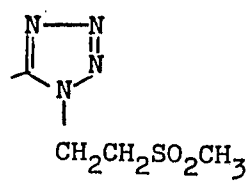 | 1760 1660 1600 | 3,0(s,3H), 3,45(m,2H), 3,8 (m,2H), 4,2(m,2H), 4,7 (m,2H), 4,85 (d,1H), 5,4 (s,1H), 5,6(d,1H), 6,7 (d,2H), 7,3(d,2H), 7,7 (d,2H), 8,0(d,2H), 8,37 (s,1H). |

Die Verbindungen der allgemeinen Formel I und I' lassen sich in die üblichen pharmazeutischen Anwendungsformen, wie Tabletten, Dragées, Kapseln oder Ampullen einarbeiten. Die Einzeldosis beträgt bei Erwachsenen im allgemeinen zwischen 50 und 1 000 mg, vorzugsweise 100 bis 500 mg, die Tagesdosis zwischen 100 und 4 000 mg, vorzugsweise 250 bis 2 000 mg.

Beispiel I

Tabletten enthaltend Natrium-7-{D-α-[3-(2-p-aminosulfonylanilino-4-hydroxy-5-pyrimidinyl)-ureido]-p-hydroxyphenylacetamido}-3-[(1-vinyl-tetrazol-5-yl)-thiomethyl]-ceph-3-em-4-carboxylat

Ein Gemisch bestehend aus 2 kg Wirksubstanz, 5 kg Lactose, 1,8 kg Kartoffelstärke, 0,1 kg Magnesiumstearat und 0,1 kg Talk wird in üblicher Weise zu Tabletten gepreßt, derart, daß jede Tablette 200 mg Wirkstoff enthält.

Beispiel II

Dragées enthaltend Natrium-7-{D-α-[3-(2-p-aminosulfonylanilino-4-hydroxy-5-pyrimidinyl)-ureido]-p-hydroxyphenylacetamido}-3-[(1-vinyl-tetrazol-5-yl)-thiomethyl]-ceph-3-em-4-carboxylat

Analog Beispiel I werden Tabletten gepreßt, die anschließend in üblicher Weise mit einem Überzug bestehend aus Zucker, Kartoffelstärke, Talk und Tragant überzogen werden.

Beispiel III

Kapseln enthaltend Natrium-7-{D-α-[3-(2-p-aminosulfonylanilino-4-hydroxy-5-pyrimidinyl)-ureido]-p-hydroxyphenylacetamido}-3-[(1-vinyl-tetrazol-5-yl)-thiomethyl]-ceph-3-em-4-carboxylat

5 kg Wirksubstanz werden in üblicher Weise in Hartgelatinekapseln gefüllt, derart, daß jede Kapsel 500 mg des Wirkstoffes enthält.

Beispiel IV

Trockenampullen enthaltend Natrium-7-{D-α-[3(2-p-aminosulfonylanilino-4-hydroxy-5-pyrimidinyl)-ureido]-p-hydroxyphenylacetamido}-3-[(1-vinyl-tetrazol-5-yl)-thiomethyl]-ceph-3-em-4-carboxylat

In einem aseptischen Bereich wurde 251 g Wirkstoff in 20 ml destilliertem Wasser zur Injektion aufgelöst. Die Lösung wurde durch ein Millipore-Filter (Porengröße 0,22 um, Produkt der Millipore Corporation, Bedford, USA) filtriert. Die Lösung wurde jeweils in einer Menge von 2,0 ml in 1 000 Gläschen (Kapazität 10 ml) eingegossen und es wurde lyophilisiert. Die Gläschen wurde sodann mit einem Kautschukstöpsel und einer Aluminiumkappe verschlossen. Somit wurden Gläschen (Nr. A) jeweils mit 250 mg Wirkstoff erhalten.

Eine physiologische Kochsalzlösung zur Injektion wurde in einer Menge von jeweils 2,0 ml in Ampullen abgefüllt und die Ampullen wurden verschlossen. Auf diese Weise wurden Ampullen (Nr. B) erhalten. Die physiologische Kochsalzlösung in den Ampullen (Nr. B) wurde in die Gläschen (Nr. A) gegossen, wodurch eine injizierbare Zubereitung für die intravenöse Verabreichung erhalten wurde.

Destilliertes Wasser zur Injektion wurde in einer Menge von 20 ml in die Gläschen (Nr. A) gegossen und die Lösung wurde in einer 5%igen Lösung von Glucose für Injektionen (250 ml) aufgelöst. Auf diese Weise wurden Lösungen für die kontinuierliche Infusion hergestellt.

Analog sind Tabletten, Dragées, Kapseln und Ampullen erhältlich, die einen oder mehrere der übrigen Wirkstoffe der Formel I oder die physiologisch unbedenklichen Salze dieser Verbindungen enthalten.

**Ansprüche** (für die Vertragsstaaten : BE, CH, DE, FR, IT, LI, LU, NL, SE)

1. Cephalosporine der allgemeinen Formeln

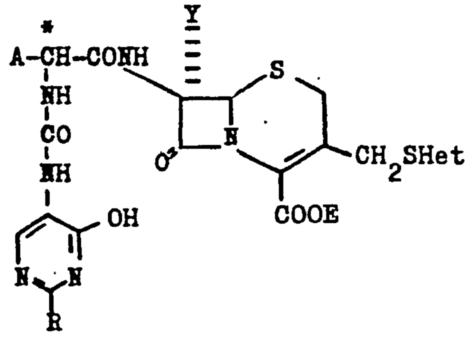

(I)

bzw.

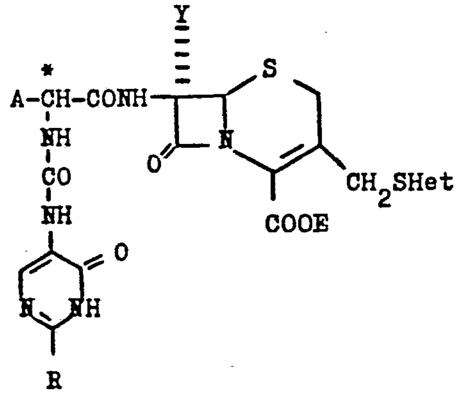

(I')

in welchen A, E, R, Y und Het die folgenden Bedeutungen besitzen :

A die Phenyl-, 4-Hydroxyphenyl-, 2-Thienyl-, 2-Furylgruppe oder einen 3,4-Dihydroxyphenylrest,

Y ein Wasserstoffatom oder die Methoxygruppe,

Het die 4H-5,6-Dioxo-1,2,4-triazin-3-yl-, die 4-Methyl-5,6-dioxo-1,2,4,triazin-3-yl-, die 2-Methyl-5,6-dioxo-1,2,4-triazin-3-yl-, die 1-Vinyl-tetrazol-5-yl- oder 1-Allyl-tetrazol-5-yl-gruppe oder eine Gruppe der allgemeinen Formel

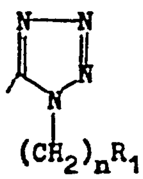

wobei n die Zahlen 1 bis 3 bedeutet und $R_1$ für die Hydroxygruppe, die Amino-, Dimethylamino-, Acetylamino-, Aminocarbonyl-, Aminocarbonylamino-, Aminosulfonyl-, Aminosulfonylamino-, Methylcarbonyl-, Methylsulfonylamino-, Cyano-, Hydroxysulfonylamino-, Methylsulfonyl-, Methylsulfinyl-, sowie für eine Carbonsäure- oder Sulfonsäuregruppe steht, weiter kann $(CH_2)_nR_1$ eine Alkylgruppe mit 2 bis 4 Kohlenstoffatomen oder einen 2,3-Dihydroxypropylrest bedeuten,

E ein Wasserstoffatom oder eine in vitro oder in vivo leicht spaltbare Schutzgruppe und

R die Cyclopropylgruppe oder eine Gruppe der allgemeinen Formel

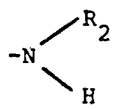 ,

worin $R_2$ eine verzweigte oder unverzweigte, gesättigte oder ungesättigte Alkylgruppe mit 1 bis 4 Kohlenstoffatomen oder einen Cycloalkylrest mit 3 bis 6 Kohlenstoffatomen bedeutet,

R bedeutet weiter eine Gruppe der allgemeinen Formel —NH—Z—G, bei der —NH—Z— eine Gruppe der Formeln

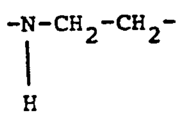 oder 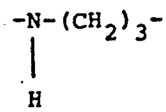

darstellt, und G die Hydroxy-, Aminocarbonyl-, Aminosulfonyl-, Aminocarbonylamino-, Acetylamino-, Methylsulfonylamino-, Methylsulfinyl- oder Methylsulfonylgruppe bedeutet oder in der

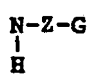

die 4'-Hydroxycyclohexylaminogruppe bedeutet ;

R bedeutet weiter eine Gruppe der allgemeinen Formel

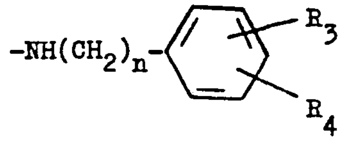

worin n = 0 oder 1 und $R_3$ und $R_4$, die gleich oder verschieden sein können, Wasserstoffatome, Chlor- oder Fluoratome, eine Hydroxy-, Methoxy-, Acetylamino-, Aminocarbonylamino-, Nitro-, Acetyl-, Methylcarbonyloxy-, Methoxycarbonyl-, Aminocarbonyl-, Cyan-, Methylsulfinyl-, Methylsulfonyl-, Aminosulfonyl-, Methylaminosulfonyl- oder eine Methylgruppe bedeuten, und falls E ein Wasserstoffatom bedeutet, deren physiologisch verträglichen Salze mit anorganischen oder organischen Basen.

2. Cephalosporine der allgemeinen Formeln I bzw. I' gemäß Anspruch 1, dadurch gekennzeichnet, daß

A die Phenyl-, p-Hydroxyphenyl-, 3,4-Dihydroxyphenyl-, 2-Thienyl- oder 2- Furylgruppe und

E ein Wasserstoffatom bedeuten und

Y, R und Het wie in Anspruch 1 definiert sind, und deren physiologisch verträglichen Salze mit anorganischen oder organischen Basen.

3. Cephalosporine der allgemeinen Formeln I bzw. I' gemäß Anspruch 1, dadurch gekennzeichnet, daß R die Gruppe der allgemeinen Formel

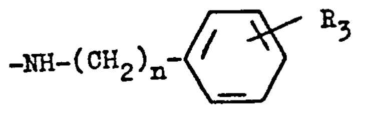

bedeutet, worin n = 0 oder 1 ist und $R_3$ ein Wasserstoffatom, ein Chloratom, die Hydroxy-, Nitro-, Acetylamino-, Methylsulfinyl-, Methylsulfonyl-, Acetyl-, Aminocarbonyl-, Aminocarbonylamino, Aminosulfonyl- oder Methylaminosulfonylgruppe bedeutet, oder daß R die p-Aminosulfonyl-m-hydroxy-anilinogruppe oder die Cyclopropyl-, Propylamino-, Isopropylamino-, Cyclopentylamino-, Cyclohexylamino-, 3'-Hydroxy-propylamino-, 4'-Hydroxycyclohexylamino- oder 2'-Aminosulfonyläthylaminogruppe darstellt und A, E, Y und Het wie im Anspruch 1 definiert sind, und, falls E ein Wasserstoffatom darstellt, deren physiologisch verträgliche Salze mit anorganischen oder organischen Basen.

4. Cephalosporine der allgemeinen Formeln I bzw. I' gemäß den Ansprüchen 1 bis 3, dadurch gekennzeichnet, daß A, R, Y und Het die in den Ansprüchen 1, 2 oder 3 angegebenen Bedeutungen besitzen und E als leicht abspaltbare Gruppe die Benzyl-, Diphenylmethyl-, Trityl-, t-Butyl-, die 2,2,2-Trichloräthyl-, die Trimethylsilylgruppe oder eine Alkanoyloxyalkylgruppe mit 1 bis 5 Kohlenstoffatomen im Alkanoylrest und mit 1 bis 3 Kohlenstoffatomen im Alkylenrest oder die Phthalidyl- oder Indanylgruppe darstellt.

5. a) Natrium-7-{D-α-[3-(2-p-aminosulfonylanilino-4-hydroxy-5-pyrimidinyl)-ureido]-p-hydroxyphenylacetamido}-3-[(1-vinyl-tetrazol-5-yl)-thiomethyl]-ceph-3-em-4-carboxylat

b) Natrium-7-{D-α-[3-(2-p-aminosulfonylanilino-4-hydroxy-5-pyrimidinyl)-ureido]-p-hydroxyphenylacetamido}-3-[(1-allyl-tetrazol-5-yl)-thiomethyl]-ceph-3-em-4-carboxylat

c) Natrium-7-{D-α-[3-(2-p-aminosulfonylanilino-4-hydroxy-5-pyrimidinyl)-ureido]-p-hydroxyphenylacetamido}-3-[(4-methyl-5,6-dioxo-1,2,4-triazin-3-yl)-thiomethyl]-ceph-3-em-4-carboxylat

d) Natrium-7-{D-α-[3-(2-p-aminosulfonylanilino-4-hydroxy-5-pyrimidinyl)-ureido]-p-hydroxyphenylacetamido}-3-[(1-(2'-dimethylaminoäthyl)-tetrazol-5-yl)-thiomethyl]-ceph-3-em-4-carboxylat

e) 7-{D-α-[3-(2-p-Aminosulfonylanilino-4-hydroxy-5-pyrimidinyl)-ureido]-p-hydroxyphenylacetamido}-3-[(1-carboxymethyl-tetrazol-5-yl)-thiomethyl]-ceph-3-em-4-carbonsäure-Di-Natriumsalz

f) Natrium-7-{D-α-[3-(4-hydroxy-2-(4'-hydroxycyclohexylamino)-5-pyrimidinyl)-ureido]-p-hydroxyphenylacetamido}-3-[(1-vinyl-tetrazol-5-yl)-thiomethyl]-ceph-3-em-4-carboxylat

g) Natrium-7-{D-α-[3-(2-p-aminosulfonylanilino-4-hydroxy-5-pyrimidinyl)-ureido]-p-hydroxyphenylacetamido}-3-[(1-(2'-hydroxyäthyl)-tetrazol-5-yl)-thiomethyl]-ceph-3-em-4-carboxylat

h) Natrium-7ß-{D-α-[3-(2-p-aminosulfonylanilino-4-hydroxy-5-pyrimidinyl)-ureido]-p-hydroxyphenylacetamido}-7α-methoxy-3-[(1-vinyl-tetrazol-5-yl)-thiomethyl]-ceph-3-em-4-carboxylat

6. Arzneimittel gekennzeichnet durch einen Gehalt an einem oder mehreren Wirkstoffen der allgemeinen Formeln I oder I' gemäß den Ansprüchen 1 bis 5 neben der üblichen Träger- und/oder Hilfsstoffen.

7. Verfahren zur Herstellung Cephalosporine der allgemeinen Formel

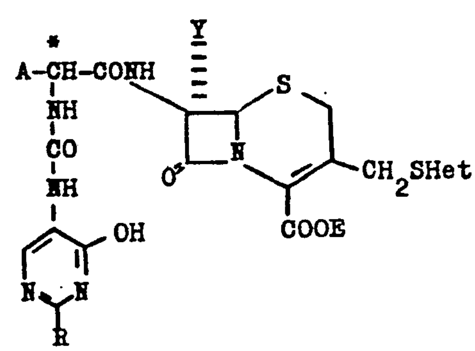

(I)

bzw.

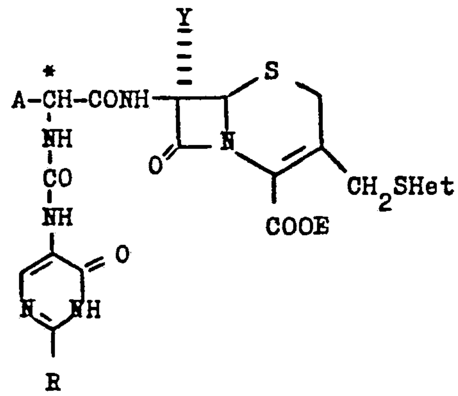

(I')

in welchen A, E, R, Y und Het die folgenden Bedeutungen besitzen :

A die Phenyl-, 4-Hydroxyphenyl-, 2-Thienyl-, 2-Furylgruppe oder einen 3,4-Dihydroxyphenylrest,

Y ein Wasserstoffatom oder die Methoxygruppe,

Het die 4H-5,6-Dioxo-1,2,4-triazin-3-yl-, die 4-Methyl-5,6-dioxo-1,2,4-triazin-3-yl-, die 2-Methyl-5,6-dioxo-1,2,4-triazin-3-yl-, die 1-Vinyl-tetrazol-5-yl- oder 1-Allyl-tetrazol-5-yl-gruppe oder eine Gruppe der allgemeinen Formel

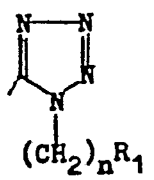

wobei n die Zahlen 1 bis 3 bedeutet und $R_1$ für die Hydroxygruppe, die Amino-, Dimethylamino-, Acetylamino-, Aminocarbonyl-, Aminocarbonylamino-, Aminosulfonyl-, Aminosulfonylamino-, Methylcarbonyl-, Methylsulfonylamino-, Cyano-, Hydroxysulfonylamino-, Methylsulfonyl-, Methylsulfinyl-, sowie für eine Carbonsäure- oder Sulfonsäuregruppe steht, weiter kann $(CH_2)_nR_1$ eine Alkylgruppe mit 2 bis 4 Kohlenstoffatomen oder einen 2,3-Dihydroxypropylrest bedeuten,

E ein Wasserstoffatom oder eine in vitro oder in vivo leicht spaltbare Schutzgruppe und

R die Cyclopropylgruppe oder eine Gruppe der allgemeinen Formel

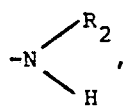 ,

worin $R_2$ eine verzweigte oder unverzweigte, gesättigte oder ungesättigte Alkylgruppe mit 1 bis 4 Kohlenstoffatomen oder einen Cycloalkylrest mit 3 bis 6 Kohlenstoffatomen bedeutet,

R bedeutet weiter eine Gruppe der allgemeinen Formel —NH—Z—G, bei der —NH—Z eine Gruppe der Formeln

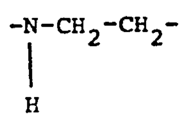 oder 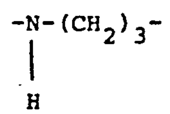

darstellt, und G die Hydroxy-, Aminocarbonyl-, Aminosulfonyl-, Aminocarbonylamino-, Acetylamino-, Methylsulfonylamino-, Methylsulfinyl- oder Methylsulfonylgruppe bedeutet oder in der

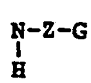

die 4'-Hydroxycyclohexylaminogruppe bedeutet ;

R bedeutet weiter eine Gruppe der allgemeinen Formel

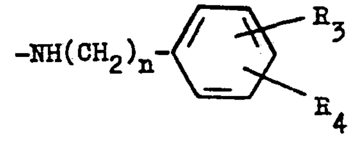

worin n = 0 oder 1 und $R_3$ und $R_4$, die gleich oder verschieden sein können, Wasserstoffatome, Chlor- oder Fluoratome, eine Hydroxy-, Methoxy-, Acetylamino-, Aminocarbonylamino-, Nitro-, Acetyl-,

Methylcarbonyloxy-, Methoxycarbonyl-, Aminocarbonyl-, Cyan-, Methylsulfinyl-, Methylsulfonyl-, Aminosulfonyl-, Methylaminosulfonyl- oder eine Methylgruppe bedeuten, und, falls E ein Wasserstoffatom bedeutet, von deren physiologisch verträglichen Salzen mit anorganischen oder organischen Basen, dadurch gekennzeichnet, daß

a) eine Verbindung der allgemeinen Formel II,

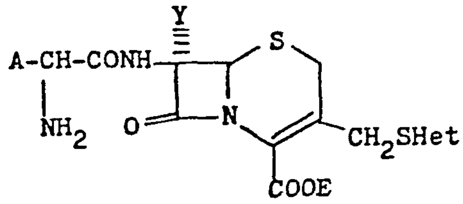

(II)

in der A, Y, E und Het die oben angegebenen Bedeutungen haben, mit einem Pyrimidinderivat der allgemeinen Formel III,

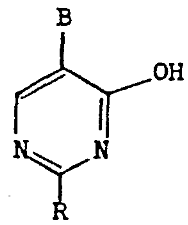

(III)

in der R wie oben definiert ist und B die Gruppen —NCO, —NHCOCl, —NHCOBr oder

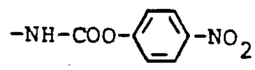

bedeutet, oder mit Gemischen solcher Pyrimidinderivaten der allgemeinen Formel III, in der B teils die eine und teils die andere der vorstehend genannten Bedeutungen besitzt, in einem Lösungsmittel und bei einem pH-Bereich zwischen 2,0 und 9,0 bei Temperaturen zwischen − 20 °C und + 50 °C umgesetzt wird, oder

b) eine Ureidocarbonsäure der allgemeinen Formel IV,

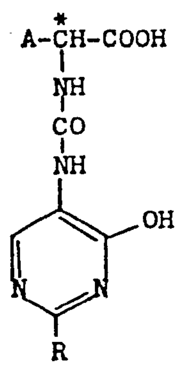

(IV)

in der A und R die oben angegebenen Bedeutungen besitzen, oder ihre Salze oder reaktiven Derivate, mit 7-Aminocephalosporansäurederivaten der allgemeinen Formel V,

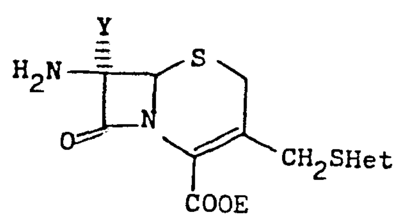

(V)

in der E, Y und Het wie oben definiert sind, zwischen − 40° und + 40 °C in Gegenwart eines Lösungsmittels und gegebenenfalls in Gegenwart einer Base umgesetzt wird, oder

c) zur Herstellung von Cephalosporinderivaten der allgemeinen Formeln I bzw. I', in der E ein Wasserstoffatom bedeutet, eine Verbindung der allgemeinen Formel VI,

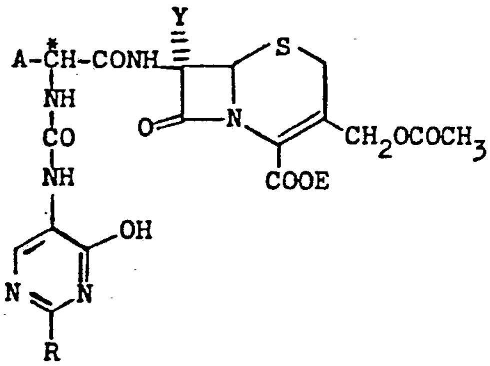

(VI)

in der A, R und Y die oben angegebenen Bedeutungen haben und E Wasserstoff bedeutet, mit einer Verbindung der allgemeinen Formel VII,

Het—S—M, (VII)

in der Het die oben angegebenen Bedeutungen hat und M für ein Wasserstoffatom oder ein Alkalimetall oder ein Erdalkalimetall steht, in einem organischen Lösungsmittel oder in Wasser oder in Gemischen dieser Lösungsmittel bei einem pH-Wert der Reaktionslösung zwischen 2 und 10, vorteilhafterweise zwischen 4 und 8, bei einer Temperatur im Bereich von 0° bis 100 °C umgesetzt wird und/oder, gegebenenfalls anschließend, eine nach den vorstehend genannten Verfahren hergestellte Verbindung der allgemeinen Formel I bzw. I', in der Y ein Wasserstoffatom darstellt, in Anwesenheit von Methanol mit einem Alkalimetall-Methylat der allgemeinen Formel $M^+OCH_3^-$, worin $M^+$ ein Alkalimetallkation bedeutet, und mit einem Halogenierungsmittel in einem inerten Lösungsmittel bei Temperaturen zwischen − 120° und − 10 °C zu einer Verbindung der allgemeinen Formeln I bzw. I', in denen Y die Methoxygruppe bedeutet, umgesetzt wird und/oder eine erhaltene Verbindung der allgemeinen Formeln I oder I', in denen E für eine in vitro leicht abspaltbare Schutzgruppe steht, in die freie Carbonsäure der allgemeinen Formeln I bzw. I', in der E ein Wasserstoffatom bedeutet, überführt wird, und/oder eine Verbindung der allgemeinen Formeln I oder I', in der E ein Wasserstoffatom bedeutet, in ihre Ester oder, mittels anorganischen oder organischen Basen, in die entsprechenden Salze übergeführt wird.

8. Verfahren gemäß Anspruch 7a, dadurch gekennzeichnet, daß eine Verbindung der allgemeinen Formel II, in der E Wasserstoff bedeutet, oder eines ihrer Salze mit anorganischen oder organischen Basen mit einer Verbindung der allgemeinen Formel III,

a) in Wasser oder in mit Wasser mischbaren Lösungsmitteln in Gegenwart von Wasser in einem pH-Bereich von 6,5 bis 8,0 oder

b) in wasserfreien Lösungsmitteln oder

c) in einem Gemenge aus Wasser und mit Wasser nicht mischbaren Lösungsmitteln in einem pH-Bereich zwischen 6,5- und 8,0

umgesetzt wird, oder daß eine Verbindung der allgemeinen Formel II, in der E eine Silylgruppe oder eine andere leicht abspaltbare Schutzgruppe bedeutet, mit einer Verbindung der allgemeinen Formel III in einem wasser- und hydroxylgruppenfreien bzw. aprotischen Lösungsmittel, gegebenenfalls in Gegenwart einer Base, umgesetzt wird.

9. Verfahren gemäß Anspruch 7b, dadurch gekennzeichnet, daß als reaktive Derivate der Ureidocarbonsäuren der allgemeinen Formeln IV deren Säureanhydride, deren reaktive Ester oder reaktive Amide oder deren Säurehalogenide, als reaktive Ester der Verbindungen der allgemeinen Formel V der Diphenylmethylester, der t-Butylester, der Trimethylsilylester oder das N,O-Bis-trimethylsilylderivat verwendet werden und die Umsetzung in Gegenwart einer Base und/oder eines organischen Lösungsmittels und/oder eines Kondensationsmittels erfolgt und, gewünschtenfalls, das so erhaltene Produkt der allgemeinen Formel I, in der E eine abspaltbare Schutzgruppe bedeutet, anschließend in eine Verbindung der allgemeinen Formel I übergeführt wird, in der E ein Wasserstoffatom darstellt.

10. Verfahren gemäß Anspruch 7 zur Herstellung einer Verbindung der allgemeinen Formel I oder I', worin E den Pivaloyloxymethylrest bedeutet, dadurch gekennzeichnet, daß eine Verbindung der allgemeinen Formel I oder I', in der E ein Wasserstoffatom darstellt, in ihr Alkalisalz übergeführt und dieses mit einem Pivaloyloxymethylhalogenid der allgemeinen Formel

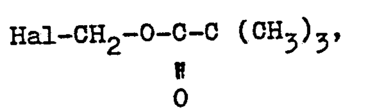

worin Hal für Chlor, Brom oder Jod steht, in einem Lösungsmittel bei Temperaturen zwischen 20 und 45 °C umgesetzt wird.

**Ansprüche** (für den Vertragsstaat AT)

1. Verfahren zur Herstellung von Cephalosporinen der allgemeinen Formel (I)

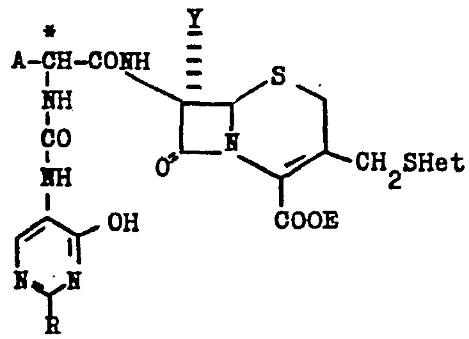

(I)

bzw.

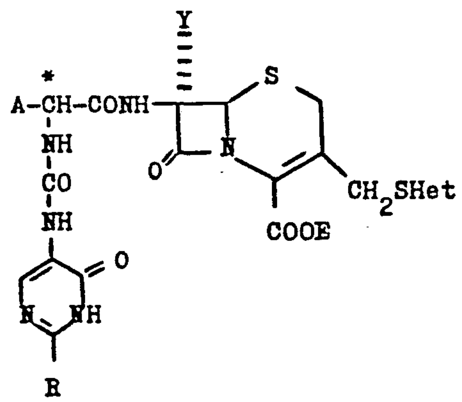

(I')

in welchen A, E, R, Y und Het die folgenden Bedeutungen besitzen :

A die Phenyl-, 4-Hydroxyphenyl-, 2-Thienyl-, 2-Furylgruppe oder einen 3,4-Dihydroxyphenylrest,

Y ein Wasserstoffatom oder die Methoxygruppe,

Het die 4H-5,6-Dioxo-1,2,4-triazin-3-yl-, die 4-Methyl-5,6-dioxo-1,2,4-triazin-3-yl-, die 1-Vinyl-tetrazol-5-yl- oder 1-Allyl-tetrazol-5-yl-gruppe oder eine Gruppe der allgemeinen Formel

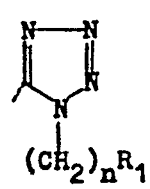

wobei n die Zahlen 1 bis 3 bedeutet und $R_1$ für die Hydroxygruppe, die Amino-, Dimethylamino-, Acetylamino-, Aminocarbonyl-, Aminocarbonylamino-, Aminosulfonyl-, Aminosulfonylamino-, Methylcarbonyl-, Methylsulfonylamino-, Cyano-, Hydroxysulfonylamino-, Methylsulfonyl-, Methylsulfinyl-, sowie für eine Carbonsäure- oder Sulfonsäuregruppe steht, weiter kann $(CH_2)_nR_1$ eine Alkylgruppe mit 2 bis 4 Kohlenstoffatomen oder einen 2,3-Dihydroxypropylrest bedeuten,

E ein Wasserstoffatom oder eine in vitro oder in vivo leicht spaltbare Schutzgruppe und

R die Cyclopropylgruppe oder eine Gruppe der allgemeinen Formel

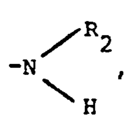

worin $R_2$ eine verzweigte oder unverzweigte, gesättigte oder ungesättigte Alkylgruppe mit 1 bis 4 Kohlenstoffatomen oder einen Cycloalkylrest mit 3 bis 6 Kohlenstoffatomen bedeutet,

R bedeutet weiter eine Gruppe der allgemeinen Formel —NH—Z—G, bei der —NH—Z eine Gruppe der Formeln

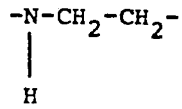 oder 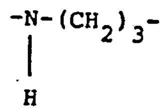

darstellt, und G die Hydroxy-, Aminocarbonyl-, Aminosulfonyl-, Aminocarbonylamino-, Acetylamino-, Methylsulfonylamino-, Methylsulfinyl- oder Methylsulfonylgruppe bedeutet oder in der

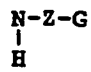

die 4'-Hydroxycyclohexylaminogruppe bedeutet ;

R bedeutet weiter eine Gruppe der allgemeinen Formel

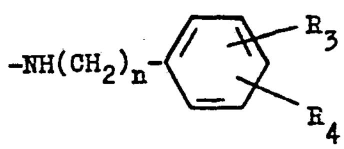

worin n = 0 oder 1 und $R_3$ und $R_4$, die gleich oder verschieden sein können, Wasserstoffatome, Chlor- oder Fluoratome, eine Hydroxy-, Methoxy-, Acetylamino-, Aminocarbonylamino-, Nitro-, Acetyl-, Methylcarbonyloxy-, Methoxycarbonyl-, Aminocarbonyl-, Cyan-, Methylsulfinyl-, Methylsulfonyl-, Aminosulfonyl-, Methylaminosulfonyl- oder eine Methylgruppe bedeuten,
und, falls E ein Wasserstoffatom bedeutet, von deren physiologisch verträglichen Salzen mit anorganischen oder organischen Basen, dadurch gekennzeichnet, daß

a) eine Verbindung der allgemeinen Formel II,

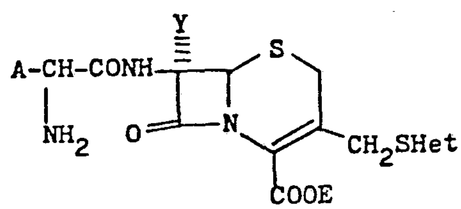 (II)

in der A, Y, E und Het die oben angegebenen Bedeutungen haben, mit einem Pyrimidinderivat der allgemeinen Formel III,

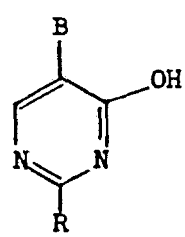 (III)

in der R wie oben definiert ist und B die Gruppen —NCO, —NHCOCl, —NHCOBr oder

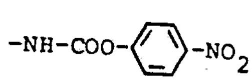

bedeutet, oder mit Gemischen solcher Pyrimidinderivaten der allgemeinen Formel III, in der B teils die eine und teils die andere der vorstehend genannten Bedeutungen besitzt, in einem Lösungsmittel und bei einem pH-Bereich zwischen 2,0 und 9,0 bei Temperaturen zwischen − 20 °C und + 50 °C umgesetzt wird, oder

b) eine Ureidocarbonsäure der allgemeinen Formel IV,

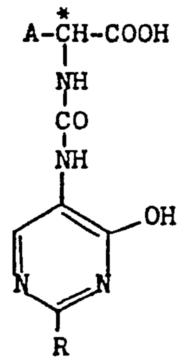

(IV)

in der A und R die oben angegebenen Bedeutungen besitzen, oder ihre Salze oder reaktiven Derivate, mit 7-Amino-cephalosporansäurederivaten der allgemeinen Formel V,

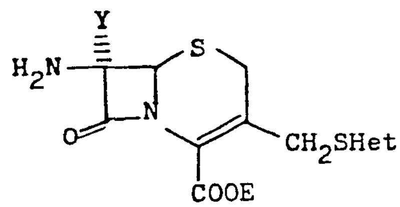

(V)

in der E, Y und Het wie oben definiert sind, zwischen − 40 °C und + 40 °C in Gegenwart eines Lösungsmittels und gegebenenfalls in Gegenwart einer Base umgesetzt wird, oder

c) zur Herstellung von Cephalosporinderivaten der allgemeinen Formeln I bzw. I', in der E ein Wasserstoffatom bedeutet, eine Verbindung der allgemeinen Formel VI,

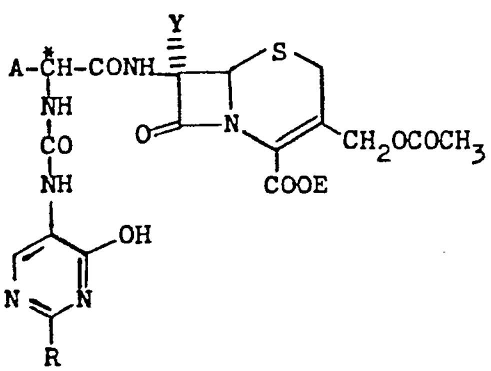

(VI)

in der A, R und Y die oben angegebenen Bedeutungen haben und E Wasserstoff bedeutet, mit einer Verbindung der allgemeinen Formel VII,

Het—S—M, (VII)

in der Het die oben angegebenen Bedeutungen hat und M für ein Wasserstoffatom oder ein Alkalimetall oder ein Erdalkalimetall steht, in einem organischen Lösungsmittel oder in Wasser oder in Gemischen dieser Lösungsmittel bei einem pH-Wert der Reaktionslösung zwischen 2 und 10, vorteilhafterweise zwischen 4 und 8, bei einer Temperatur im Bereich von 0° bis 100 °C umgesetzt wird und/oder, gegebenenfalls anschließend, eine nach den vorstehend genannten Verfahren hergestellte Verbindung der allgemeinen Formel I bzw. I', in der Y ein Wasserstoffatom darstellt, in Anwesenheit von Methanol mit einem Alkalimetall-Methylat der allgemeinen Formel $M^+OCH_3^-$, worin $M^+$ ein Alkalimetallkation bedeutet, und mit einem Halogenierungsmittel in einem inerten Lösungsmittel bei Temperaturen zwischen − 120° und − 10 °C zu einer Verbindung der allgemeinen Formeln I bzw. I', in denen Y die Methoxygruppe bedeutet, umgesetzt wird und/oder eine erhaltene Verbindung der allgemeinen Formeln I oder I', in

denen E für eine in vitro leicht abspaltbare Schutzgruppe steht, in die freie Carbonsäure der allgemeinen Formeln I bzw. I', in der E ein Wasserstoffatom bedeutet, überführt wird, und/oder eine Verbindung der allgemeinen Formeln I oder I', in der E ein Wasserstoffatom bedeutet, in ihre Ester oder, mittels anorganischen oder organischen Basen, in die entsprechenden Salze übergeführt wird.

2. Verfahren zur Herstellung von Cephalosporinen der allgemeinen Formel I bzw. I' gemäß Anspruch 1, in welchen A, R, Y und E die im Anspruch 1 genannten Bedeutungen besitzen und Het die 2-Methyl-5,6-dioxo-1,2,4-triazin-3-ylgruppe darstellt und, falls E ein Wasserstoffatom bedeutet, von deren physiologisch verträglichen Salzen mit anorganischen oder organischen Basen, dadurch gekennzeichnet, daß diese Verbindungen nach den im Anspruch 1 genannten Verfahren hergestellt werden.

3. Verfahren gemäß Anspruch 1a und 2, dadurch gekennzeichnet, daß eine Verbindung der allgemeinen Formel II, in der E Wasserstoff bedeutet, oder eines ihrer Salze mit anorganischen oder organischen Basen mit einer Verbindung der allgemeinen Formel III,

a) in Wasser oder in mit Wasser mischbaren Lösungsmitteln in Gegenwart von Wasser in einem pH-Bereich von 6,5 bis 8,0 oder

b) in wasserfreien Lösungsmitteln oder

c) in einem Gemenge aus Wasser und mit Wasser nicht mischbaren Lösungsmitteln in einem pH-Bereich zwischen 6,5- und 8,0

umgesetzt wird, oder daß eine Verbindung der allgemeinen Formel II, in der E eine Silylgruppe oder eine andere leicht abspaltbare Schutzgruppe bedeutet, mit einer Verbindung der allgemeinen Formel III in einem wasser- und hydroxylgruppenfreien bzw. aprotischen Lösungsmittel, gegebenenfalls in Gegenwart einer Base, umgesetzt wird.

4. Verfahren gemäß Anspruch 1b und 2, dadurch gekennzeichnet, daß als reaktive Derivate der Ureidocarbonsäuren der allgemeinen Formeln IV deren Säureanhydride, deren reaktive Ester oder reaktive Amide oder deren Säurehalogenide, als reaktive Ester der Verbindungen der allgemeinen Formel V der Diphenylmethylester, der t-Butylester, der Trimethylsilylester oder das N,O-Bis-trimethylsilylderivat verwendet werden und die Umsetzung in Gegenwart einer Base und/oder eines organischen Lösungsmittels und/oder eines Kondensationsmittels erfolgt und, gewünschtenfalls, das so erhaltene Produkt der allgemeinen Formel I, in der E eine abspaltbare Schutzgruppe bedeutet, anschließend in eine Verbindung der allgemeinen Formel I übergeführt wird, in der E ein Wasserstoffatom darstellt.

5. Verfahren gemäß Anspruch 1 und 2 zur Herstellung einer Verbindung der allgemeinen Formel I oder I', worin E den Pivaloyloxymethylrest bedeutet, dadurch gekennzeichnet, daß eine Verbindung der allgemeinen Formel I oder I', in der E ein Wasserstoffatom darstellt, in ihr Alkalisalz übergeführt und dieses mit einem Pivaloyloxymethylhalogenid der allgemeinen Formel

$$Hal-CH_2-O-\underset{\underset{\displaystyle O}{\|}}{C}-C\,(CH_3)_3,$$

worin Hal für Chlor, Brom oder Jod steht, in einem Lösungsmittel bei Temperaturen zwischen 20 und 45 °C umgesetzt wird.

**Claims** (for the Contracting States : BE, CH, DE, FR, IT, LI, LU, NL, SE)

1. Cephalosporins corresponding to the general formulae

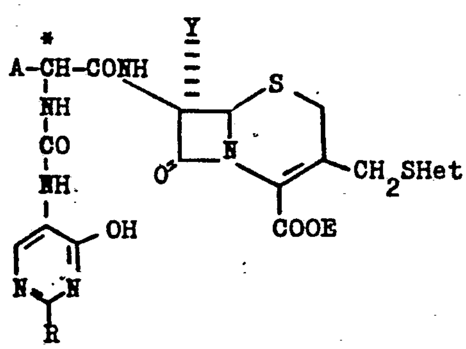

(I)

and

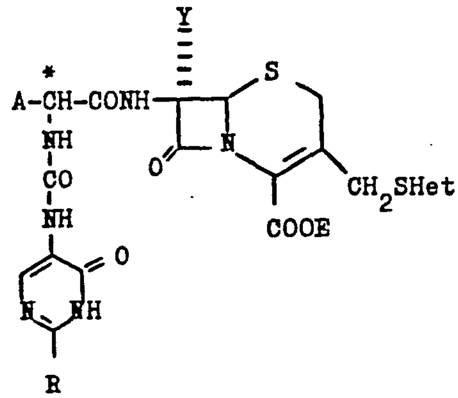

(I')

wherein A, E, R, Y and Het have the following meanings :

A represents the phenyl, 4-hydroxyphenyl, 2-thienyl, 2-furyl group or a 3,4-dihydroxyphenyl radical

Y represents a hydrogen atom or the methoxy group,

Het represents the 4H-5,6-dioxo-1,2,4-triazin-3-yl, 4-methyl-5,6-dioxo-1,2,4-triazin-3-yl, 2-methyl-5,6-dioxo-1,2,4-triazin-3-yl, 1-vinyl-tetrazol-5-yl or 1-allyl-tetrazol-5-yl group or a group corresponding to the general formula

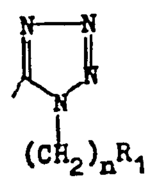

in which n represents the integers 1 to 3 and $R_1$ represents the hydroxy group, the amino, dimethylamino, acetylamino, aminocarbonyl, aminocarbonylamino, aminosulphonyl, aminosulphonylamino, methylcarbonyl, methylsulphonylamino, cyano, hydroxysulphonylamino, methylsulphonyl, methylsulphinyl group or a carboxylic acid or sulphonic acid group, moreover, $(CH_2)_nR_1$ can represent an alkyl group with 2 to 4 carbon atoms or a 2,3-dihydroxypropyl radical

E represents a hydrogen atom or a protective group which can readily be split in vitro or in vivo and

R represents the cyclopropyl group or a group corresponding to the general formula

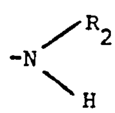 ,

wherein $R_2$ represents a branched or unbranched, saturated or unsaturated alkyl group with 1 to 4 carbon atoms or a cycloalkyl radical with 3 to 6 carbon atoms,

R also represents a group corresponding to the general formula —NH—Z—G, in which —NH—Z— denotes a group corresponding to the formulae

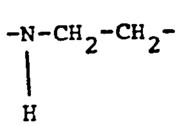 or 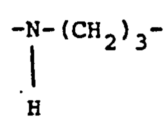

and G represents the hydroxy, aminocarbonyl, aminosulphonyl, aminocarbonylamino, acetylamino, methylsulphonylamino, methylsulphinyl or methylsulphonyl group or in which

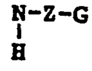

represents the 4'-hydroxycyclohexylamino group ;

R also represents a group corresponding to the general formula

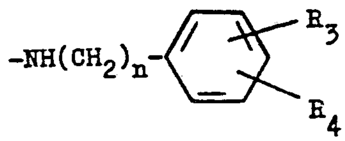

wherein n = 0 or 1 and $R_3$ and $R_4$, which may be the same or different, represent hydrogen atoms, chlorine or fluorine atoms, a hydroxy, methoxy, acetylamino, aminocarbonylamino, nitro, acetyl, methylcarbonyloxy, methoxycarbonyl, aminocarbonyl, cyano, methylsulphinyl, methylsulphonyl, aminosulphonyl, methylaminosulphonyl or methyl group, and, if E represents a hydrogen atom, the physiologically compatible salts thereof with inorganic or organic bases.

2. Cephalosporins corresponding to the general formulae I and I' according to claim 1, characterised in that

A represents the phenyl, p-hydroxyphenyl, 3,4-dihydroxyphenyl, 2-thienyl or 2-furyl group and

E represents a hydrogen atom and

Y, R and Het are as defined in claim 1, and the physiologically compatible salts thereof with inorganic or organic bases.

3. Cephalosporins corresponding to the general formulae I and I' according to claim 1, characterised in that R represents the group corresponding to the general formula

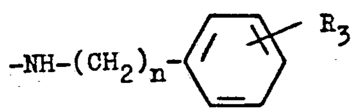

wherein n = 0 or 1 and $R_3$ represents a hydrogen atom, a chlorine atom, the hydroxy, nitro, acetylamino, methylsulphinyl, methylsulphonyl, acetyl, aminocarbonyl, aminocarbonylamino, aminosulphonyl or methylaminosulphonyl group or R represents the p-aminosulphonyl-m-hydroxy-anilino group or the cyclopropyl, propylamino, isopropylamino, cyclopentylamino, cyclohexylamino, 3'-hydroxy-propylamino, 4'-hydroxycyclohexylamino or 2'-aminosulphonylethylamino group and A, E, Y and Het are as defined in claim 1, and, if E represents a hydrogen atom, the physiologically compatible salts thereof with inorganic or organic bases.

4. Cephalosporins corresponding to the general formulae I and I' according to claims 1 to 3, characterised in that A, R, Y and Het have the meanings given in claims 1, 2 or 3 and E, as a readily splittable group, represents the benzyl, diphenylmethyl, trityl, t-butyl, 2,2,2-trichloroethyl, trimethylsilyl group or an alkanoyloxyalkyl group with 1 to 5 carbon atoms in the alkanoyl radical and with 1 to 3 carbon atoms in the alkylene radical or the phthalidyl or indanyl group.

5. a) Sodium 7-{D-α-[3-(2-p-aminosulphonylanilino-4-hydroxy-5-pyrimidinyl)-ureido]-p-hydroxyphenylacetamido}-3-[(1-vinyl-tetrazol-5-yl)-thiomethyl]-ceph-3-em-4-carboxylate

b) Sodium 7-{D-α-[3-(2-p-aminosulphonylanilino-4-hydroxy-5-pyrimidinyl)-ureido]-p-hydroxyphenylacetamido}-3-[(1-allyl-tetrazol-5-yl)-thiomethyl]-ceph-3-em-4-carboxylate

c) Sodium 7-{D-α-[3-(2-p-aminosulphonylanilino-4-hydroxy-5-pyrimidinyl)-ureido]-p-hydroxyphenylacetamido}-3-[(4-methyl-5,6-dioxo-1,2,4-triazin-3-yl)-thiomethyl]-ceph-3-em-4-carboxylate

d) Sodium 7-{D-α-[3-(2-p-aminosulphonylanilino-4-hydroxy-5-pyrimidinyl)-ureido]-p-hydroxyphenylacetamido}-3-[(1-(2'-dimethylaminoethyl)-tetrazol-5-yl)-thiomethyl]-ceph-3-em-4-carboxylate

e) 7-{D-α-[3-(2-p-aminosulphonylanilino-4-hydroxy-5-pyrimidinyl)-ureido]-p-hydroxyphenylacetamido}-3-[(1-carboxymethyl-tetrazol-5-yl)-thiomethyl]-ceph-3-em-4-carboxylic acid disodium salt

f) Sodium 7-{D-α-[3-(4-hydroxy-2-(4'-hydroxycyclohexylamino)-5-pyrimidinyl)-ureido]-p-hydroxyphenylacetamido}-3-[(1-vinyl-tetrazol-5-yl)-thiomethyl]-ceph-3-em-4-carboxylate

g) Sodium 7-{D-α-[3-(2-p-aminosulphonylanilino-4-hydroxy-5-pyrimidinyl)-ureido]-p-hydroxyphenylacetamido}-3-[(1-(2'-hydroxyethyl)-tetrazol-5-yl)-thiomethyl]-ceph-3-em-4-carboxylate

h) Sodium 7β-{D-α-[3-(2-p-aminosulphonylanilino-4-hydroxy-5-pyrimidinyl)-ureido]-p-hydroxyphenylacetamido}-7α-methoxy-3-[(1-vinyltetrazol-5-yl)-thiomethyl]-ceph-3-em-4-carboxylate.

6. Drugs characterised by a content of one or more active ingredients corresponding to general formula I or I' according to claims 1 to 5 in addition to the conventional carriers and/or auxiliaries.

7. A process for the preparation of cephalosporins corresponding to the general formula

(See Figure page 39)

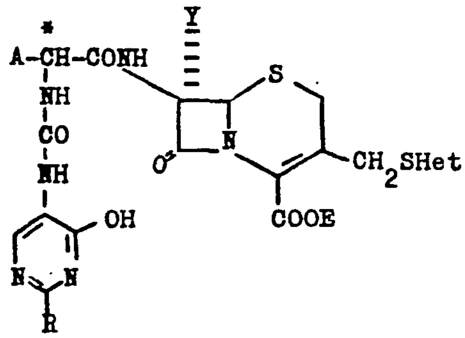

(I)

and

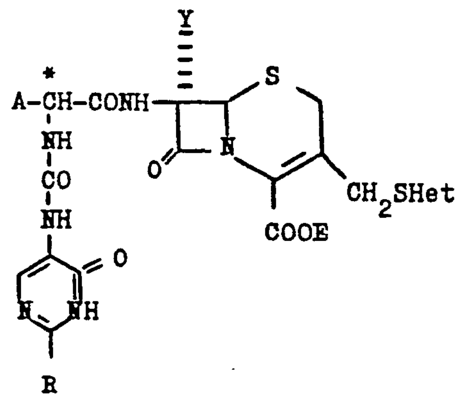

(I')

in which A, E, R, Y and Het have the following meanings :

A represents the phenyl, 4-hydroxyphenyl, 2-thienyl, 2-furyl group or a 3,4-dihydroxyphenyl radical,

Y represents a hydrogen atom or the methoxy group,

Het represents the 4H-5,6-dioxo-1,2,4-triazin-3-yl-, 4-methyl-5,6-dioxo-1,2,4-triazin-3-yl, 2-methyl-5,6-dioxo-1,2,4-triazin-3-yl, 1-vinyl-tetrazol-5-yl or 1-allyl-tetrazol-5-yl group or a group corresponding to the general formula

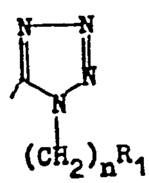

in which n represents the integers 1 to 3 and $R_1$ represents the hydroxy group, the amino, dimethylamino, acetylamino, aminocarbonyl, aminocarbonylamino, aminosulphonyl, aminosulphonylamino, methycarbonyl, methylsulphonylamino, cyano, hydroxysulphonylamino, methylsulphonyl, methylsulphinyl group or a carboxylic acid or sulphonic acid group, moreover, $(CH_2)_nR_1$ can be an alkyl group with 2 to 4 carbon atoms or a 2,3-dihydroxypropyl radical

E represents a hydrogen atom or a protective group which is readily splittable in vitro or in vivo and

R represents the cyclopropyl group or a group corresponding to the general formula

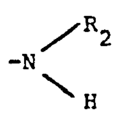 ,

wherein $R_2$ denotes a branched or unbranched, saturated or unsaturated alkyl group with 1 to 4 carbon atoms or a cycloalkyl radical with 3 to 6 carbon atoms,

R also represents a group corresponding to the general formula —NH—Z—G, in which —NH—Z represents a group corresponding to the formulae

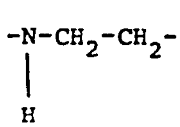 or 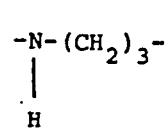

and G represents the hydroxy, aminocarbonyl, aminosulphonyl, aminocarbonylamino, acetylamino, methylsulphonylamino, methylsulphinyl or methylsulphonyl group or in which

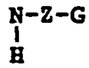

represents the 4′-hydroxycyclohexylamino group ;

R also represents a group corresponding to the general formula

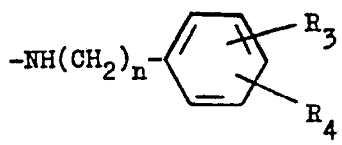

wherein n = 0 or 1 and $R_3$ and $R_4$, which may be the same or different, represent hydrogen atoms, chlorine or fluorine atoms, a hydroxy, methoxy, acetylamino, aminocarbonylamino, nitro, acetyl, methylcarbonyloxy, methoxycarbonyl, aminocarbonyl, cyano, methylsulphinyl, methylaminosulphonyl or a methyl group, and, if E denotes a hydrogen atom, the physiologically compatible salts thereof with inorganic or organic bases, characterised in that

a) a compound corresponding to the general formula II

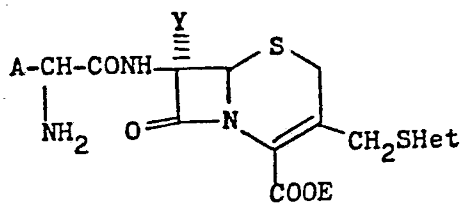

(II)

in which A, Y, E and Het have the meanings given above, is reacted with a pyrimidine derivative corresponding to the general formula III

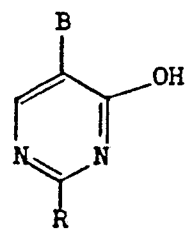

(III)

in which R is as defined above and B represents the groups —NCO, —NHCOCl, —NHCOBr or

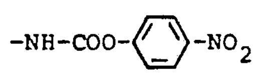

or with mixtures of these pyrimidine derivatives corresponding to general formula III, in which B partly has one of the meanings given above and partly the other, in a solvent and at a pH range between 2.0 and 9.0 at temperatures between − 20 °C and + 50 °C, or

b) a ureidocarboxylic acid corresponding to the general formula IV

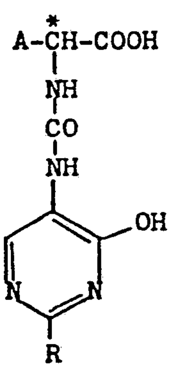

(IV)

in which A and R have the meanings given above, or salts or reactive derivatives thereof, is reacted with 7-aminocephalosporanic acid derivatives corresponding to the general formula V,

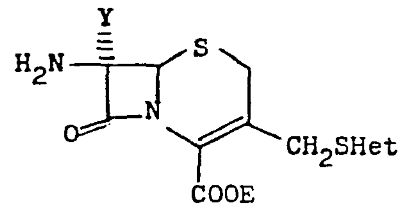

(V)

in which E, Y and Het are as defined above, between – 40° and + 40 °C in the presence of a solvent and optionally in the presence of a base, or

c) for the production of cephalosporin derivatives corresponding to general formula I and I', in which E represents a hydrogen atom, a compound corresponding to the general formula VI

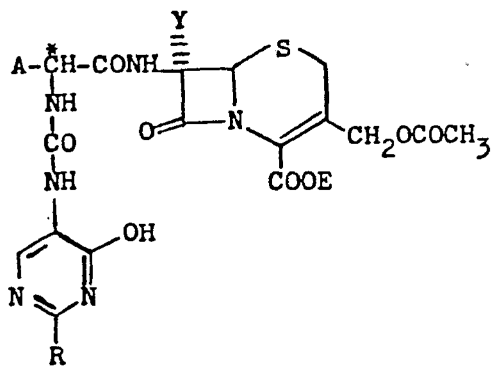

(VI)

in which A, R and Y have the meanings given above and E represents hydrogen, is reacted with a compound corresponding to the general formula VII

Het—S—M (VII)

in which Het has the meanings given above and M represents a hydrogen atom or an alkaline earth metal or an alkali metal, in an organic solvent or in water or in mixtures of these solvents at a pH value of the reaction solution of between 2 and 10, advantageously between 4 and 8, at a temperature in the range from 0° to 100 °C and/or, optionally afterwards, a compound prepared by the above-mentioned process corresponding to general formula I and I', in which Y represents a hydrogen atom, is reacted in the presence of methanol with an alkali metal methoxide corresponding to the general formula $M^+OCH_3^-$, in which $M^+$ represents an alkali metal cation, and with a halogenation agent in an inert solvent at temperatures between – 120° and – 10 °C to form a compound corresponding to general formula I or I', in which Y represents the methoxy group, and/or a resultant compound corresponding to general formula I or I', in which E represents a protective group readily splittable in vitro, is converted into the free carboxylic acid corresponding to general formulae I and I', in which E represents a hydrogen atom, and/or a compound corresponding to general formula I and I', in which E represents a hydrogen atom, is converted into esters thereof or, using inorganic or organic bases, into the corresponding salts.

8. A process according to claim 7a, characterised in that a compound corresponding to general formula II, in which E represents hydrogen or one of its salts with inorganic or organic bases is reacted with a compound corresponding to the general formula III

a) in water or in solvents miscible with water in the presence of water in a pH range from 6.5 to 8.0 or

b) in solvents free from water or

c) in a mixture of water and solvents immiscible with water in a pH range between 6.5 and 8.0 or that a compound corresponding to the general formula II in which E represents a silyl group or a different readily splittable protective group, is reacted with a compound corresponding to general formula III in a water-free and hydroxyl group-free or aprotic solvent, optionally in the presence of a base.

9. A process according to claim 7b, characterised in that, as reactive derivatives of ureidocarboxylic acids corresponding to the general formula IV there are used the acid anhydrides, reactive esters or reactive amides or acid halides thereof, and as reactive esters of the compounds corresponding to the formula V there are used the diphenylmethyl ester, t-butyl ester, trimethylsilyl ester or N,O-bis-

trimethylsilyl derivative, and the reaction takes place in the presence of a base and/or a condensation agent and, if desired, the resultant product corresponding to the general formula I, in which E represents a splittable protective group, is subsequently converted into a compound corresponding to general formula I in which E represents a hydrogen atom.

10. A process according to claim 7 for the production of a compound corresponding to the general formula I or I', in which E represents the pivaloyloxymethyl radical, characterised in that a compound corresponding to the general formula I or I', in which E represents a hydrogen atom, is converted into the alkali salt thereof and the latter is reacted with a pivaloxymethyl halide corresponding to the general formula

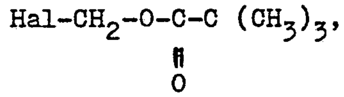

wherein Hal represents chlorine, bromine or iodine, in a solvent at temperatures between 20 and 45 °C.

**Claims** (for the Contracting State AT)

1. A process for the production of cephalosporins corresponding to the general formula

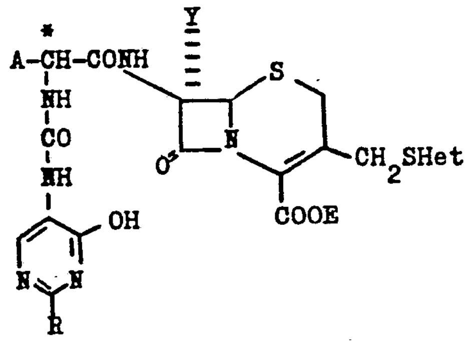

(I)

and

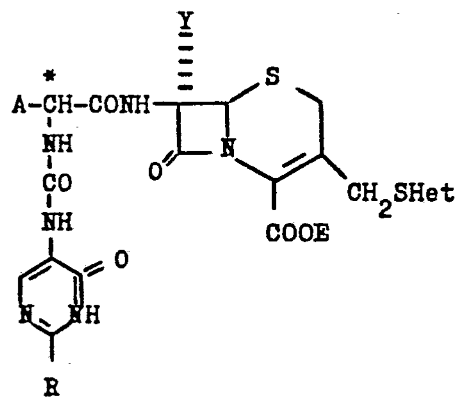

(I')

in which A, E, R, Y and Het have the following meanings :

A represents the phenyl, 4-hydroxyphenyl, 2-thienyl, 2-furyl group or a 3,4-dihydroxyphenyl radical,

Y represents a hydrogen atom or the methoxy group,

Het represents the 4H-5,6-dioxo-1,2,4-triazin-3-yl, 4-methyl-5,6-dioxo-1,2,4-triazin-3-yl, 1-vinyl-tetrazol-5-yl or 1-allyl-tetrazol-5-yl group or a group corresponding to the general formula

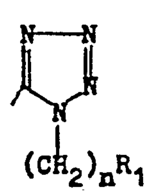

in which n represents the integers 1 to 3 and $R_1$ represents the hydroxy group, the amino, dimethylamino, acetylamino, aminocarbonyl, aminocarbonylamino, aminosulphonyl, aminosulphonylamino, methylcarbonyl, methylsulphonylamino, cyano, hydroxysulphonylamino, methylsulphonyl, methylsulphinyl, or a carboxylic acid or sulphonic acid group, moreover, $(CH_2)R_1$ can represent an alkyl group with 2 to 4 carbon atoms or a 2,3-dihydroxypropyl radical

E represents a hydrogen atom or a protective group readily splittable in vitro or in vivo

R represents the cyclopropyl group or a group corresponding to the general formula

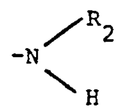

wherein $R_2$ represents a branched or unbranched, saturated or unsaturated alkyl group with 1 to 4 carbon atoms or a cycloalkyl radical with 3 to 6 carbon atoms,

R also represents a group corresponding to the general formula —NH—Z—G, in which —NH—Z denotes a group corresponding to the formulae

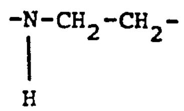 or 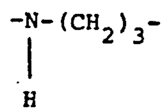

and G represents the hydroxy, aminocarbonyl, aminosulphonyl, aminocarbonylamino, acetylamino, methylsulphonylamino, methylsulphinyl or methylsulphonyl group or in which

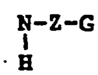

denotes the 4'-hydroxycyclohexylamino group

R also represents a group corresponding to the general formula

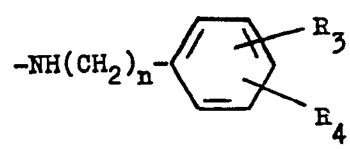

wherein n = 0 or 1 and $R_3$ and $R_4$, which may be the same or different, represent hydrogen atoms, chlorine or fluorine atoms, a hydroxy, methoxy, acetylamino, aminocarbonylamino, nitro, acetyl, methylcarbonyloxy, methoxycarbonyl, aminocarbonyl, cyano, methylsulphinyl methylsulphonyl, aminosulphonyl, methylaminosulphonyl or a methyl group and, if E represents a hydrogen atom, the physiologically compatible alts thereof with inorganic or organic bases, characterised in that

a) a compound corresponding to the general formula II

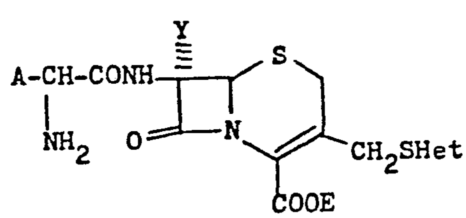

(II)

in which A, Y, E and Het have the meanings given above is reacted with a pyrimidine derivative corresponding to the general formula III

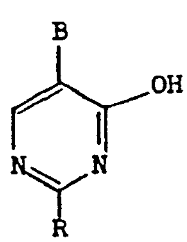

(III)

in which R is as defined above and B represents the groups —NCO, —NHCOCl, —NHCOBr or

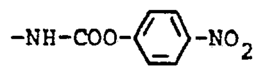

or with mixtures of these pyrimidine derivatives corresponding to general formula III, in which B partly has one of the meanings given above and partly the other, in a solvent and in a pH range between 2.0 and 9.0 at temperatures between − 20 °C and + 50 °C, or

b) a ureidocarboxylic acid corresponding to general formula IV

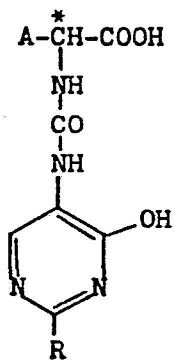

(IV)

in which A and R have the meanings given above or salts or reactive derivatives thereof, are reacted with 7-amino-cephalosporanic acid derivatives corresponding to the general formula V,

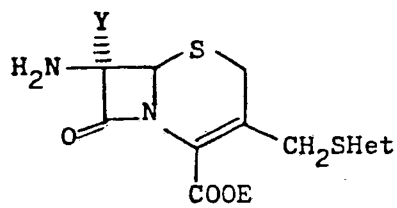

(V)

in which E, Y and Het are as defined above, at between − 40° and + 40 °C in the presence of a solvent and optionally in the presence of a base, or

c) for the production of cephalosporin derivatives corresponding to general formulae I and I', in which E represents a hydrogen atom, a compound corresponding to the general formula VI,

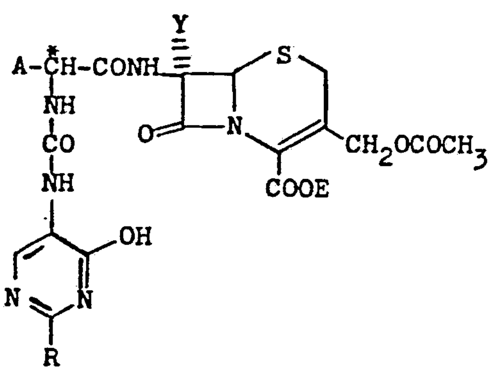

(VI)

in which A, R and Y have the meanings given above and E represents hydrogen, is reacted with a compound corresponding to general formula VII

Het—S—M (VII)

in which Het has the meanings given above and M represents a hydrogen atom or an alkali metal or an alkaline earth metal, in an organic solvent or in water or in mixtures of these solvents at a pH value of the

reaction solution of between 2 and 10, advantageously between 4 and 8, at a temperature in the range from 0° to 100 °C and/or, optionally afterwards, a compound produced by the above-mentioned process corresponding to general formula I and I′, in which Y represents a hydrogen atom, is reacted in the presence of methanol with an alkali metal methoxide corresponding to the general formula $M^+OCH_3^-$, wherein $M^+$ denotes an alkali metal cation, and with a halogenation agent in an inert solvent at temperatures between − 120° and − 10 °C forming a compound corresponding to the general formulae I and I′, in which Y represents the methoxy group and/or a resultant compound corresponding to the general formula I or I′, in which E represents a protective group readily splittable in vitro, is converted into the free carboxylic acid corresponding to the general formulae I and I′, in which E represents a hydrogen atom, and/or a compound corresponding to the general formula I or I′, in which E represents a hydrogen atom, is converted into its esters or, using inorganic or organic bases, into the corresponding salts.

2. A process for the production of cephalosporins corresponding to the general formula I or I′ according to claim 1, in which A, R, Y and E have the meanings given in claim 1 and Het represents the 2-methyl-5,6-dioxo-1,2,4-triazin-3-yl group and, if E denotes a hydrogen atom, the physiologically compatible salts thereof with inorganic or organic bases, characterised in that these compounds are produced by the process mentioned in claim 1.

3. A process according to claim 1a and 2, characterised in that a compound corresponding to the general formula II, in which E represents hydrogen, or one of its salts with inorganic or organic bases, is reacted with a compound corresponding to the general formula III,

a) in water or in solvents miscible with water in the presence of water in a pH range of 6.5 to 8.0 or

b) in water-free solvents, or

c) in a mixture of water and solvents immiscible with water in a pH range between 6.5 and 8.0 or that a compound corresponding to the general formula II, in which E represents a silyl group or a different readily splittable protective group, is reacted with a compound corresponding to the general formula III in a water-free and hydroxyl group-free or aprotic solvent, optionally in the presence of a base.

4. A process according to claim 1b and 2, characterised in that as reactive derivatives of ureidocarboxylic acids corresponding to the general formula IV, there are used the acid anhydrides, reactive esters or reactive amides or acid halides thereof, as reactive esters of the compounds corresponding to the general formula V, there are used the diphenylmethyl esters, t-butyl esters, trimethylsilyl ester or the N,O-bis-trimethylsilyl derivative, and the reaction takes place in the presence of a base and/or of an organic solvent and/or of a condensation agent and, if desired, the resultant product corresponding to the general formula I, in which E represents a splittable protective group, in then converted into a compound corresponding to the general formula I, in which E represents a hydrogen atom.

5. A process according to claim 1 and 2 for the production of a compound corresponding to the general formula I or I′, wherein E represents the pivaloyloxymethyl radical, characterised in that a compound corresponding to general formula I or I′, in which E represents a hydrogen atom, is converted into its alkali salt and this is reacted with a pivaloyloxymethyl halide corresponding to the general formula

$$Hal{-}CH_2{-}O{-}\underset{\underset{O}{\|}}{C}{-}C\ (CH_3)_3,$$

wherein Hal represents chlorine, bromine or iodine, in a solvent at temperatures between 20 and 45 °C.

**Revendications** (pour les Etats contractants : BE, CH, DE, FR, IT, LI, LU, NL, SE)

1. Céphalosporines de formules générales

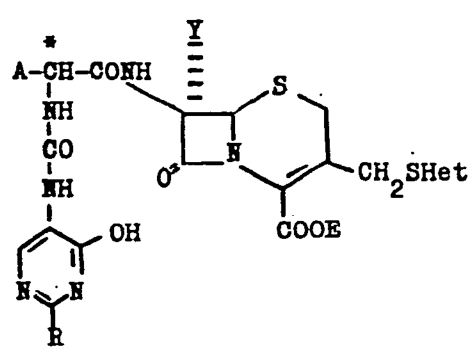

(I)

ou respectivement

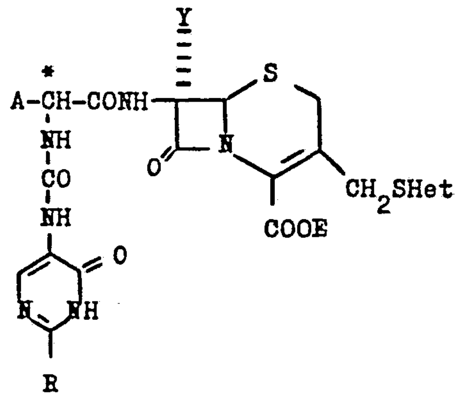

(I')

dans lesquelles A, E, R, Y et Het possèdent les significations suivantes

A représente le groupe phényle, 4-hydroxyphényle, 2-thiényle, 2-furyle ou un radical 3,4-dihydroxyphényle

Y représente un atome d'hydrogène ou le groupe méthoxy,

Het représente le groupe 4H-5,6-dioxo-1,2,4-triazine-3-yle, 4-méthyl-5,6-dioxo-1,2,4, triazine-3-yle, 2-méthyl-5,6-dioxo-1,2,4-triazine-3-yle, 1-vinyl-tétrazol-5-yle ou 1-allyl-tétrazol-5-yle ou un groupe de formule générale

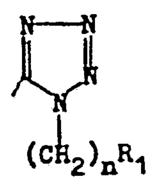

n représentant les nombres 1 à 3 et $R_1$ remplaçant le groupe hydroxy, le groupe amino diméthylamino acétylamino, aminocarbonyle, aminocarbonylamino, aminosulfonyle, aminosulfonylamino, méthylcarbonyle, méthylsulfonylamino, cyano, hydroxysulfonylamino, méthylsulfonylamino, cyano, hydroxysulfonylamino, méthylsulfonyle, méthylsulfinyle, ainsi qu'un groupe acide carboxylique ou acide sulfonique de plus $(CH_2)_nR^1$ peut représenter un groupe alcoyle avec 2 à 4 atomes de carbone ou un radical 2,3-dihydroxypropyle

E représente un atome d'hydrogène ou un groupe protecteur facilement clivable in vitro ou in vivo et

R représente le groupe cyclopropyle ou un groupe de formule générale

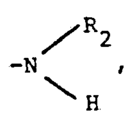

dans laquelle $R_2$ représente un groupe alcoyle avec 1 à 4 atomes de carbone saturé ou non saturé, ramifié ou non ramifié ou un radical cycloalcoyle avec 3 à 6 atomes de carbone.

R représente en outre un groupe de formule générale —NH—Z—G dans laquelle —NH—Z— représente un groupe selon les formules

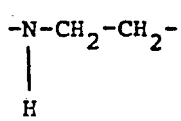

ou

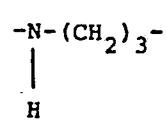

et G représente le groupe hydroxy, aminocarbonyle, aminosulfonyle, aminocarbonylamino, acétylamino, méthylsulfonylamino, méthylsulfinyle ou méthysulfonyle ou dans laquelle

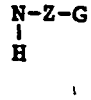

représente le groupe 4'-hydroxycyclohexylamino ;

R représente en outre un groupe de formule générale

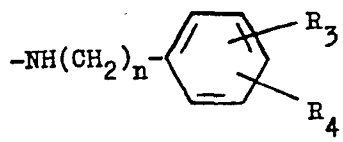

dans laquelle n = 0 ou 1 et $R_3$ et $R_4$, qui peuvent être identiques ou différents, représentent des atomes d'hydrogène, des atomes de chlore ou de fluor, un groupe hydroxy, méthoxy, acétylamino, aminocarbonylamino, nitro, acétyle, méthylcarbonyloxy, méthoxycarbonyle, aminocarbonyle, cyano, méthylsulfinyle, méthylsulfonyle, aminosulfonyle, méthylaminosulfonyle ou méthyle, et lorsque E représente un atome d'hydrogène, leurs sels physiologiquement supportables avec des bases minérales ou organiques.

2. Céphalosporines de formules générales I ou respectivement I, selon la revendication 1, caractérisées en ce que

A représente le groupe phényle, p-hydroxyphényle, 3,4-dihydroxyphényle, 2-thiényle ou 2-furyle et E représente un atome d'hydrogène et

Y, R et Het sont définis comme dans la revendication 1, et leurs sels physiologiquement supportables avec des bases minérales ou organiques.

3. Céphalosporines de formules générales I ou respectivement I' selon la revendication 1, caractérisées en ce que R représente le groupe de formule générale

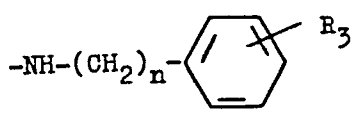

dans laquelle n = 0 ou 1 et $R_3$ représente un atome d'hydrogène, un atome de chlore, le groupe hydroxy, nitro, acétylamino, méthylsulfinyle, méthylsulfonyle, acétyle, aminocarbonyle, aminocarbonylamino, aminosulfonyle ou méthylaminosulfonyle, ou en ce que R représente le groupe p-aminosulfonyl-m-hydroxy-anilino ou le groupe cyclopropyle, propylamino, ispopropylamino, cyclopentylamino, cyclohexylamino, 3'-hydroxy-propylamino, 4'-hydroxycyclohexylamino ou 2'-aminosulfonyléthylamino et A, E, Y et Het sont définis comme dans la revendication 1, et, lorsque E représente un atome d'hydrogène, leurs sels physiologiquement supportables avec des bases minérales ou organiques.

4. Céphalosporines de formules générales I ou respectivement I' selon les revendications 1 à 3, caractérisées en ce que A, R, Y et Het possèdent les significations indiquées dans les revendications 1, 2 ou 3 et E, en tant que groupe facilement clivable, représente le groupe benzyle, diphénylméthyle, trityle, t-butyle, 2,2,2-trichloroéthyle, triméthylsilyle ou un groupe alcanoyl-oxyalcoyle avec 1 à 5 atomes de carbone dans le radical alcanoyle et avec 1 à 3 atomes de carbone dans le radical alcoylène ou le groupe phtalidyle ou indanyle.

5. a) le 7-{D-α-[3-(2-p-aminosulfonylanilino-4-hydroxy-5-pyrimidinyl)-uréido]-p-hydroxyphénylacétamido}-3-[(1-vinyl-tétrazol-5-yl)-thiométhyl]-céph-3-ème-4-carboxylate de sodium.

b) le 7-{D-α-[3-(2-p-aminosulfonylanilino-4-hydroxy-5-pyrimidinyl)-uréido]-p-hydroxyphénylacétamido}-3-[(1-allyl-tétrazol-5-yl)-thiométhyl]-céph-3-ème-carboxylate de sodium.

c) le 7-{D-α-[3-(2-p-aminosulfonylanilino-4-hydroxy-5-pyrimidinyl)-uréido]-p-hydroxyphénylacétamido}-3-[(4-méthyl-5,6-dioxo-1,2,4-triazine-3-yl)-thiométhyl]-céph-3-ème-4-carboxylate de sodium.

d) le 7-{D-α-[3-(2-p-aminosulfonylanilino-4-hydroxy-5-pyrimidinyl)-uréido]-p-hydroxyphénylacétamido}-3-[(1-(2'-diméthylaminoéthyl)-tétrazol-5-yl)-thiométhyl]-céph-3-ème-4-carboxylate de sodium.

e) le sel disodique de l'acide 7-{D-α-[3-(2-p-aminosulfonylanilino-4-hydroxy-5-pyrimidinyl)-uréido]-p-hydroxyphénylacétamido}-3-[(1-carboxyméthyl-tétrazol-5-yl)-thiométhyl]-céph-3-ème-4-carboxylique.

f) le 7-{D-α-[3-(4-hydroxy-2-(4'-hydroxycyclohexylamino)-5-pyrimidinyl)-uréido]-p-hydroxyphénylacétamido}-3-[(1-vinyl-tétrazol-5-yl)-thiométhyl]-céph-3-ème-4-carboxylate de sodium.

g) le 7-{D-α-[3-(2-p-aminosulfonylanilino-4-hydroxy-5-pyrimidinyl)-uréido]-p-hydroxyphénylacétamido}-3-[(1-(2'-hydroxyéthyl)-tétrazol-5-yl)-thiométhyl]-céph-3-ème-4-carboxylate de sodium.

h) le 7β-{D-α-[3-(2-p-aminosulfonylanilino-4-hydroxy-5-pyrimidinyl)-uréido]-p-hydroxyphénylacétamido}-7-α-méthoxy-3-[(1-vinyl-tétrazol-5-yl) thiométhyl]-céph-3-ème-4-carboxylate de sodium.

6. Médicament caractérisé par une teneur en une ou plusieurs substances actives de formules générales I ou I' selon les revendications de 1 à 5, conjointement aux excipients et/ou adjuvants usuels.

7. Procédé pour la préparation de céphalosporines de formules générales I ou respectivement I'

(Voir Figure page suivante)

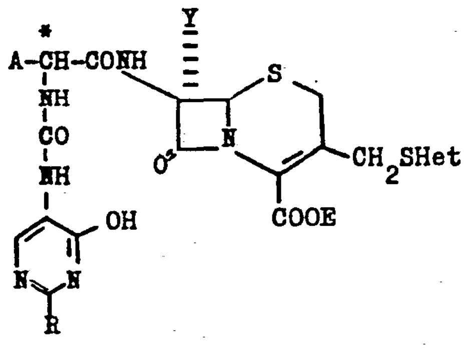

(I)

ou respectivement

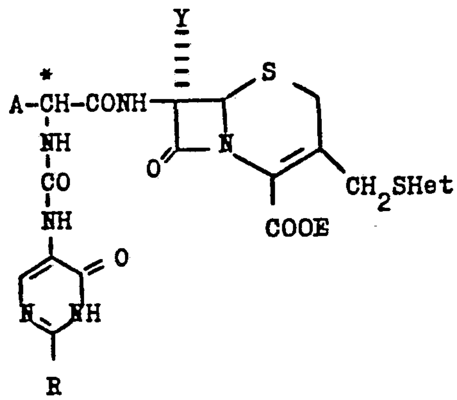

(I')

dans lesquelles A, E, R, Y et Het possèdent les significations suivantes

A représente le groupe phényle, 4-hydroxyphényle, 2-thiényle, 2-furyle ou un radical 3,4-dihydroxyphényle,

Y représente un atome d'hydrogène ou le groupe méthoxy,

Het représente le groupe 4H-5,6-dioxo-1,2,4-triazine-3-yle-, 4-méthyl-5,6-dioxo-1,2,4, triazine-3-yle, 2-méthyl-5,6-dioxo-1,2,4-triazine-3-yle, 1-vinyl-tétrazol-5-yle ou 1-allyl -tétrazol-5-yle ou un groupe de formule générale

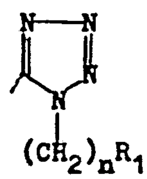

n représentant les nombres 1 à 3 et $R_1$ remplaçant le groupe hydroxy, le groupe amino, diméthylamino, acétylamino, aminocarbonyle, aminocarbonylamino, aminosulfonyle, aminosulfonylamino, méthylcarbonyle, méthylsulfonylamino, cyano, hydroxysulfonylamino, méthylsulfonyle, méthylsulfinyle, ainsi qu'un groupe acide carboxylique ou acide sulfonique, de plus $(CH_2)_nR_1$ peut représenter un groupe alcoyle avec 2 à 4 atomes de carbone ou un radical 2,3-hydroxypropyle.

E représente un atome d'hydrogène ou un groupe protecteur facilement clivable in vitro ou in vivo et

R représente le groupe cyclopropyle ou un groupe de formule générale

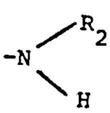,

dans laquelle $R_2$ représente un groupe alcoyle avec 1 à 4 atomes de carbone, saturé ou non saturé, ramifié ou non ramifié ou un radical cycloalcoyle avec 3 à 6 atomes de carbone.

R représente en outre un groupe de formule générale —NH—Z—G, dans laquelle —NH—Z— représente un groupe selon les formules

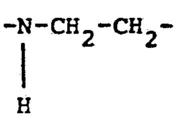

ou

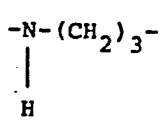

et G représente le groupe hydroxy, aminocarbonyle, aminosulfonyle, aminicarbonylamino, acétylamino, méthylsulfonylamino, méthylsulfinyle ou méthysulfonyle ou dans laquelle

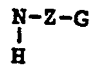

représente le groupe 4'-hydroxycyclohexylamino,

R représente en outre un groupe de formule générale

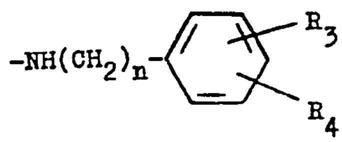

dans laquelle n = 0 ou 1 et $R_3$ et $R_4$, qui peuvent être identiques ou différents, représentent des atomes d'hydrogène, des atomes de chlore ou de fluor, un groupe hydroxy, méthoxy, acétylamino, aminocarbonylamino, nitro, acétyle, méthylcarbonyloxy, méthoxycarbonyle, aminocarbonyle, cyano, méthylsulfinyle, méthylsulfonyle, aminosulfonyle, méthylaminosulfonyle ou méthyle,

et, lorsque E représente un atome d'hydrogène, de leurs sels physiologiquement supportables avec des bases minérales ou organiques, caractérisé en ce que

a) on fait réagir un composé de formule générale II

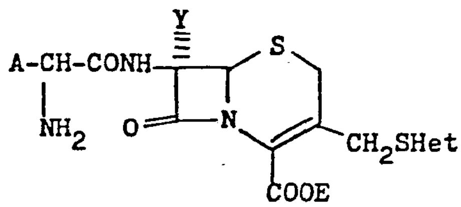

(II)

dans laquelle A, Y, E et Het ont les significations indiquées plus haut, avec un dérivé de pyrimidine de formule générale III

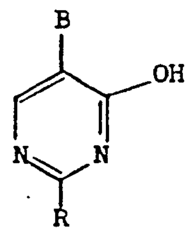

(III)

dans laquelle R est défini plus haut et B représente les groupes —NCO, —NHCOCl, —NHCOBr ou

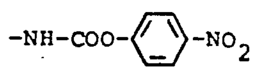

ou avec des mélanges de tels dérivés de pyrimidine de formule générale III, dans laquelle B possède en partie l'une et en partie les autres des significations mentionnées plus haut, dans un solvant et dans un domaine de pH entre 2,0 et 9,0 à des températures entre − 20 °C et + 50 °C, ou

b) on fait réagir un acide uréidocarboxylique de formule générale IV

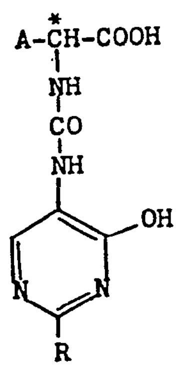

(IV)

dans laquelle A et R possèdent les significations indiquées plus haut ou ses sels ou dérivés réactifs, avec des dérivés d'acides 7-amino-céphalosporaniques de formule générale V

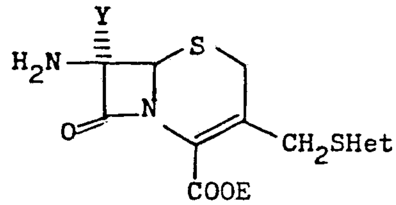

(V)

dans laquelle E, Y et Het sont définis comme plus haut, entre – 40° et + 40 °C en présence d'un solvant et éventuellement en présence d'une base ou

c) pour la préparation de dérivés de céphalosporine de formules générales I ou respectivement I', dans lesquelles E représente un atome d'hydrogène, on fait réagir un composé de formule générale VI,

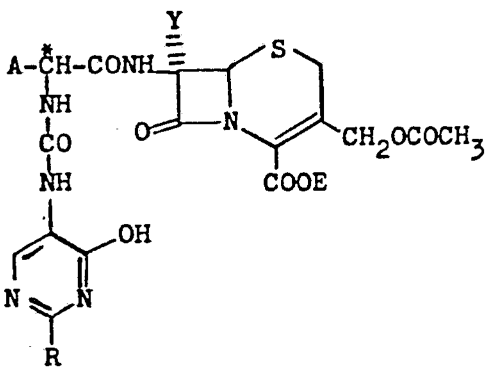

(VI)

dans laquelle A, R et Y ont les significations indiquées plus haut et E représente l'hydrogène, avec un composé de formule générale VII,

$$Het—S—M, \qquad (VII)$$

dans laquelle Het a les significations indiquées plus haut et M remplace un atome d'hydrogène ou un métal alcalin ou un métal alcalino-terreux, dans un solvant organique ou dans l'eau ou dans des mélanges de ces solvants à une valeur de pH de la solution de réaction entre 2 et 10, avantageusement entre 4 et 8, à une température dans le domaine de 0° à 100 °C et/ou, éventuellement ensuite on fait réagir un composé préparé selon l'un des procédés mentionnés plus haut de formule générale I ou I', dans laquelle Y représente un atome d'hydrogène, en présence de méthanol avec un méthylate de métal alcalin de formule générale $M^+OCH_3^-$, dans laquelle $M^+$ représente un cation de métal alcalin et avec un agent d'halogénation dans un solvant inerte à des températures entre – 120 °C et – 10 °C pour donner un composé de formules générales I ou respectivement I', dans lesquelles Y représente le groupe méthoxy et/ou on transforme un composé obtenu de formules générales I ou I' dans lesquelles E remplace un groupe protecteur facilement clivable in vitro, en l'acide carboxylique libre de formules générales I ou I', dans laquelle E représente un atome d'hydrogène, et/ou on transforme un composé de formules générales I ou I', dans laquelle E représente un atome d'hydrogène en ses esters ou, au moyen de bases minérales ou organiques, en les sels correspondants.

8. Procédé selon la revendication 7a, caractérisé en ce qu'on fait réagir un composé de formule générale II, dans laquelle E représente l'hydrogène, ou l'un de ses sels avec des bases minérales ou organiques, avec un composé de formule générale III,

a) dans l'eau ou dans des solvants miscibles à l'eau, en présence d'eau, dans un domaine de pH de 6,5 à 8,0 ou

b) dans des solvants anhydres ou

c) dans un mélange d'eau et de solvants non miscibles à l'eau dans un domaine de pH compris entre 6,5 et 8,0,

ou en ce qu'on fait réagir un composé de formule générale II, dans laquelle E représente un groupe silyle ou un autre groupe protecteur facilement clivable, avec un composé de formule générale III dans un solvant anhydre ou ne contenant pas de groupes hydroxyle ou respectivement aprotique, éventuellement en présence d'une base.

9. Procédé selon la revendication 7b, caractérisé en ce qu'on utilise en tant que dérivés réactifs, des acides uréido-carboxyliques de formule générale IV, leurs anhydrides, leurs esters réactifs ou amides réactifs, ou leurs halogénures d'acides, en tant qu'esters réactifs des composés de formule V, l'ester

diphénylméthylique, l'ester t-butylique, l'ester triméthylsylique ou le dérivé N,O-bis-triméthylsylilé et en ce que la réaction a lieu en présence d'une base et/ou d'un solvant organique et/ou d'un agent de condensation et, si on le désire, en ce que l'on transforme le produit ainsi obtenu de formule générale I dans laquelle E représente un groupe protecteur clivable, ensuite en un composé de formule générale I dans laquelle E représente un atome d'hydrogène.

10. Procédé selon la revendication 7 pour la préparation d'un composé de formule générale I ou I', dans laquelle E représente le radical pivaloyloxyméthyle, caractérisé en ce que l'on transforme un composé de formules générales I ou I' dans laquelle E représente un atome d'hydrogène, en son sel alcalin et on fait réagir celui-ci avec un halogénure de pivaloyloxyméthyle de formule générale

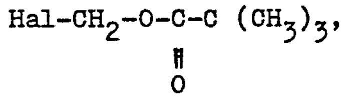

dans laquelle Hal remplace chlore, brome ou iode, dans un solvant à des températures entre 20 et 45 °C.

**Revendications** (pour l'Etat contractant AT)

1. Procédé pour la préparation de céphalosporines de formules générales I ou I'

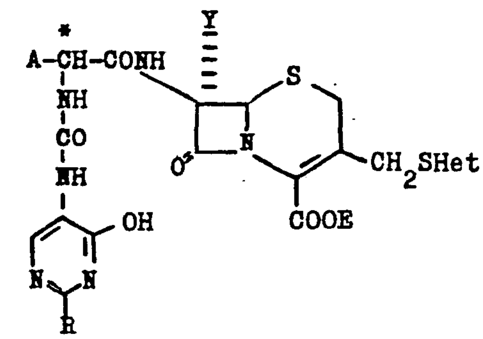

(I)

ou respectivement

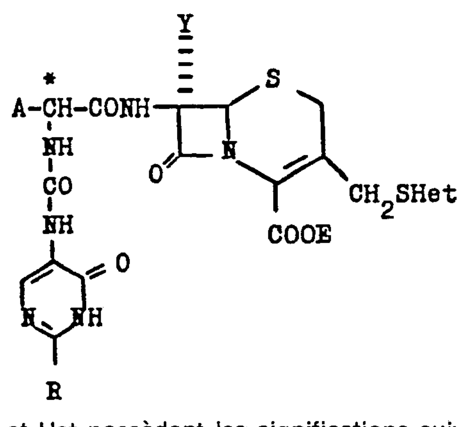

(I')

dans lesquelles A, E, R, Y et Het possèdent les significations suivantes

A représente le groupe phényle 4-hydroxyphényle, 2-thiényle, 2-furyle ou un radical 3,4-dihydroxyphényle,

Y représente un atome d'hydrogène ou le groupe méthoxy,

Het représente le groupe 4H-5,6-dioxo-1,2,4-triazine-3-yle, 4-méthyl-5,6-dioxo-1,2,4, triazine-3-yle, 2-méthyl-5,6-dioxo-1,2,4-triazine-3-yle, 1-vinyl-tétrazol-5-yle ou 1-allyl-tétrazol-5-yle ou un groupe de formule générale

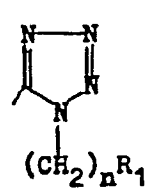

n représentant les nombres 1 à 3 et $R_1$ remplaçant le groupe hydroxy, le groupe amino, diméthylamino, acétylamino, aminocarbonyle, aminocarbonylamino, aminosulfonyle, aminosulfonylamino, méthylcarbonyle, méthylsulfonylamino, cyano, hydroxysulfonylamino, méthylsulfonyle, méthylsulfinyle, ainsi qu'un groupe acide carboxylique ou acide sulfonique, de plus $(CH_2)_nR_1$ peut représenter un groupe alcoyle avec 2 à 4 atomes de carbone ou un radical 2,3-dihydroxypropyle,

E représente un atome d'hydrogène ou un groupe protecteur facilement clivable in vitro ou in vivo et

R représente le groupe cyclopropyle ou un groupe de formule générale

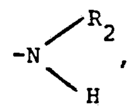

dans laquelle $R_2$ représente un groupe alcoyle avec 1 à 4 atomes de carbone, saturé ou non saturé, ramifié ou non ramifié ou un radical cycloalcoyle avec 3 à 6 atomes de carbone,

R représente en outre un groupe de formule générale —NH—Z—G, dans laquelle —NH—Z— représente un groupe selon les formules

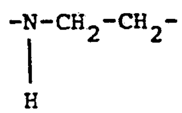 ou 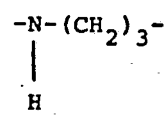

et G représente le groupe hydroxy, aminocarbonyle, aminosulfonyle, aminocarbonylamino, acétylamino, méthylsulfonylamino, méthylsulfinyle ou méthysulfonyle ou dans laquelle

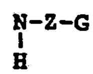

représente le groupe 4'-hydroxycyclohexylamino

R représente en outre un groupe de formule générale

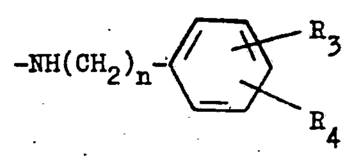

dans laquelle n = 0 ou 1 et $R_3$ et $R_4$, qui peuvent être identiques ou différents, représentent des atomes d'hydrogène, des atomes de chlore ou de fluor, un groupe hydroxy, méthoxy, acétylamino, aminocarbonylamino, nitro, acétyle, méthylcarbonyloxy, méthoxycarbonyle, aminocarbonyle, cyano, méthylsulfinyle, méthylsulfonyle, aminosulfonyle, méthylaminosulfonyle ou méthyle,

et, lorsque E représente un atome d'hydrogène, de leurs sels physiologiquement supportables avec des bases minérales ou organiques, caractérisé en ce que

a) on fait réagir un composé de formule générale II,

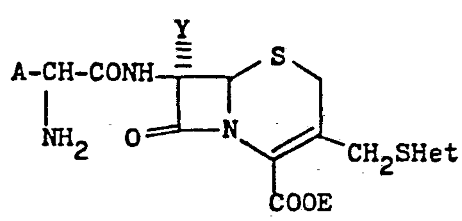

(II)

dans laquelle A, Y, E et Het ont les significations indiquées plus haut, avec un dérivé de pyrimidine de formule générale III,

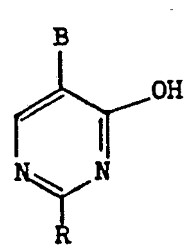

(III)

dans laquelle R est défini comme plus haut et B représente les groupes —NCO, —NHCOCl, —NHCOBr ou

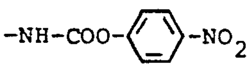

ou avec des mélanges de tels dérivés de pyrimidines de formule générale III, dans laquelle B possède en partie l'une et en partie les autres des significations mentionnées plus haut, dans un solvant et dans un domaine de pH entre 2,0 et 9,0 à des températures entre − 20° et + 50 °C, ou

b) on fait réagir un acide uréidocarboxylique de formule générale IV

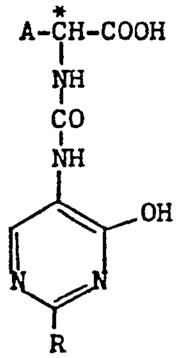

(IV)

dans laquelle A et R possèdent les significations indiquées plus haut ou ses sels ou dérivés réactifs, avec des dérivés d'acides 7-amino-céphalosporaniques de formule générale V,

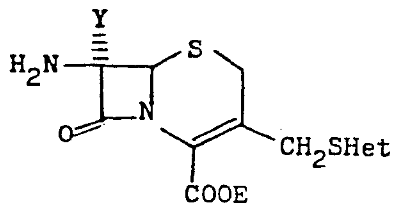

(V)

dans laquelle E, Y et Het sont définis comme plus haut, entre − 40° et + 40 °C en présence d'un solvant et éventuellement en présence d'une base ou

c) pour la préparation de dérivés de céphalosporine de formules générales I ou respectivement I', dans lesquelles E représente un atome d'hydrogène, on fait réagir un composé de formule générale VI,

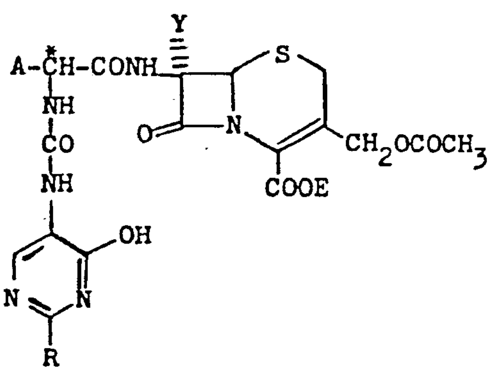

(VI)

dans laquelle A, R et Y ont les significations indiquées plus haut et E représente l'hydrogène, avec un composé de formule générale VII,

Het—S—M, (VII)

dans laquelle Het a les significations indiquées plus haut et M remplace un atome d'hydrogène ou un métal alcalin ou un métal alcalino-terreux, dans un solvant organique ou dans l'eau ou dans des mélanges de ces solvants à une valeur de pH de la solution de réaction entre 2 et 10, avantageusement entre 4 et 8, à une température dans le domaine de 0° à 100 °C et/ou,

éventuellement ensuite on fait réagir un composé préparé selon l'un des procédés mentionnés plus haut de formule générale I ou I', dans laquelle Y représente un atome d'hydrogène, en présence de méthanol avec un méthylate de métal alcalin de formule générale $M^+OCH_3^-$, dans laquelle $M^+$ représente un cation de métal alcalin, et avec un agent d'halogénation dans un solvant inerte à des températures entre − 120° et − 10 °C pour donner un composé de formules générales I ou respectivement I', dans lesquelles Y représente le groupe méthoxy et/ou on transforme un composé obtenu de formules générales I ou I' dans lesquelles E remplace un groupe protecteur facilement clivable in vitro, en l'acide carboxylique libre de formules générales I ou I', dans laquelle E représente un atome d'hydrogène, et/ou on transforme un composé de formules générales I ou I' dans laquelle E représente un atome d'hydrogène en ses esters ou, au moyen de bases minérales ou organiques, en les sels correspondants.

2. Procédé pour la préparation de céphalosporines de formule générale I ou respectivement I' selon la revendication 1, dans lesquelles A, R, Y et E possèdent les significations mentionnées dans la revendication 1 et Het représente le groupe 2-méthyl-5,6-dioxo-1,2,4-triazine-3-yle et, lorsque E représente un atome d'hydrogène, de leurs sels physiologiquement supportables avec des bases minérales ou organiques, caractérisé en ce que ces composés sont préparés selon les procédés mentionnés dans la revendication 1.

3. Procédé selon la revendication 1a et 2, caractérisé en ce qu'on fait réagir un composé de formule générale II, dans laquelle E représente l'hydrogène, ou l'un de ses sels avec des bases minérales ou organiques, avec un composé de formule générale III,

a) dans l'eau ou dans des solvants miscibles à l'eau, en présence d'eau, dans un domaine de pH de 6,5 à 8,0 ou

b) dans des solvants anhydres ou

c) dans un mélange d'eau et de solvant non miscible à l'eau dans un domaine de pH compris entre 6,5 et 8,0, ou en ce qu'on fait réagir un composé de formule générale II, dans laquelle E représente un groupe silyle ou un autre groupe protecteur facilement clivable, avec un composé de formule générale III dans un solvant anhydre ou ne contenant pas de groupes hydroxyle ou respectivement aprotique, éventuellement en présence d'une base.

4. Procédé selon la revendication 1b et 2, caractérisé en ce qu'on utilise en tant que dérivés réactifs, des acides uréido-carboxyliques de formule générale IV, leurs anhydres, leurs esters réactifs ou amides réactifs, ou leurs halogénures d'acides, en tant qu'esters réactifs des composés de formule V, l'ester diphénylméthylique, l'ester t-butylique, l'ester triméthylsilylique ou le dérivé N,O-bis-triméthylsylilé et en ce que la réaction a lieu en présence d'une base et/ou d'un solvant organique et/ou d'un agent de condensation et, si on le désire, en ce que l'on transforme le produit ainsi obtenu de formule générale I dans laquelle E représente un groupe protecteur clivable, ensuite en un composé de formule générale I dans laquelle E représente un atome d'hydrogène.

5. Procédé selon la revendication 1 et 2 pour la préparation d'un composé de formule générale I ou I', dans laquelle E représente le radical pivaloyloxyméthyle, caractérisé en ce que l'on transforme un composé de formules générales I ou I' dans laquelle E représente un atome d'hydrogène, en son sel alcalin et on fait réagir celui-ci avec un halogénure de pivaloyloxyméthyle de formule générale

$$Hal-CH_2-O-\underset{\underset{O}{\|}}{C}-C\,(CH_3)_3,$$

dans laquelle Hal remplace chlore, brome ou iode, dans un solvant à des températures entre 20 et 45 °C.